# EUROPEAN PATENT SPECIFICATION

(11) **EP 2 678 026 B1**
(45) Date of publication and mention of the grant of the patent: **18.05.2016**
(21) Application number: 12704433.7
(22) Date of filing: 21.02.2012
(51) Int. Cl.: A61K 38/17, A61K 39/00, G01N 33/53, A61P 25/28

(54) **ANKYRIN G FOR USE IN THE TREATMENT OF NEURODEGENERATIVE DISORDERS**
ANKYRIN-G ZUR VERWENDUNG IN DER BEHANDLUNG VON NEURODEGENERATIVER ERKRANKUNGEN
ANKYRINE G POUR L'UTILISATION DANS LE TRAITEMENT DE TROUBLES NEURODÉGÉNÉRATIFS

(30) Priority: 21.02.2011 EP 11001406; 22.02.2011 US 201161445447 P
(43) Date of publication of application: 01.01.2014
(73) Proprietor: The University of Zurich, 8006 Zürich (CH)
(72) Inventor: SANTUCCIONE CHADHA, Antonella, CH-8044 Zurich (CH); MERLINI, Mario, San Francisco, California 94117 (US); NITSCH, Roger, CH-8126 Zumikon (CH); GRIMM, Jan, CH-8600 Dübendorf (CH); HOCK, Christoph, CH-8703 Erlenbach (CH)
(74) Representative: Steinecke, Peter
(86) International application number: PCT/EP2012/052907
(87) International publication number: WO 2012/113775

(56) References cited:
- WO-A2-2005/098433
- GOZAL YAIR M ET AL: "Proteomics analysis reveals novel components in the detergent-insoluble subproteome in Alzheimer's disease.", JOURNAL OF PROTEOME RESEARCH NOV 2009 LNKD- PUBMED:19746990, vol. 8, no. 11, November 2009 (2009-11), pages 5069-5079, XP002637117, ISSN: 1535-3907

### SEQUENCE LISTING

<110> University of Zurich
<120> Ankyrin G and modulators thereof for the treatment of neurodegenerative disorders
<130> NE30A31-P-WO
<150> EP 11001406.5
   <151> 2001-02-21
<150> US 61/445,447
   <151> 2001-02-22
<160> 2
<170> PatentIn version 3.5
<210> 1
   <211> 4377
   <212> PRT
   <213> Homo sapiens
<300>
   <301> Kordeli E, Lambert S, Bennett V. <302> AnkyrinG. A new ankyrin gene with neural-specific isoforms localized at the axonal initial segment and node of Ranvier. <303> Journal of Biological Chemistry <304> 270 <305> 5 <306> 2352-2359 <307> 1995-02-03 <308> PMID/7836469 <309> 1995-02-03
   <313> (1) .. (4377)
<300>
   <308> UniProtKB/Q12955
   <309> 2002-07-11
   <313> (1) .. (4377)
<400> 1
   Met Ala His Ala Ala Ser Gln Leu Lys Lys Asn Arg Asp Leu Glu Ile
   1               5                   10                  15
   Asn Ala Glu Glu Glu Pro Glu Lys Lys Arg Lys His Arg Lys Arg Ser
               20                  25                  30
   Arg Asp Arg Lys Lys Lys Ser Asp Ala Asn Ala Ser Tyr Leu Arg Ala
           35                  40                  45
   Ala Arg Ala Gly His Leu Glu Lys Ala Leu Asp Tyr Ile Lys Asn Gly
       50                  55                  60
   Val Asp Ile Asn Ile Cys Asn Gln Asn Gly Leu Asn Ala Leu His Leu
   65                  70                  75                  80
   Ala Ser Lys Glu Gly His Val Glu Val Val Ser Glu Leu Leu Gln Arg
   85 90 95
   Glu Ala Asn Val Asp Ala Ala Thr Lys Lys Gly Asn Thr Ala Leu His
   100 105 110
   Ile Ala Ser Leu Ala Gly Gln Ala Glu Val Val Lys Val Leu Val Thr
   115 120 125
   Asn Gly Ala Asn Val Asn Ala Gln Ser Gln Asn Gly Phe Thr Pro Leu
   130 135 140
   Tyr Met Ala Ala Gln Glu Asn His Leu Glu Val Val Lys Phe Leu Leu
   145 150 155 160
   Asp Asn Gly Ala Ser Gln Ser Leu Ala Thr Glu Asp Gly Phe Thr Pro
   165 170 175
   Leu Ala Val Ala Leu Gln Gln Gly His Asp Gln Val Val Ser Leu Leu
   180 185 190
   Leu Glu Asn Asp Thr Lys Gly Lys Val Arg Leu Pro Ala Leu His Ile
   195 200 205
   Ala Ala Arg Lys Asp Asp Thr Lys Ala Ala Ala Leu Leu Leu Gln Asn
   210 215 220
   Asp Asn Asn Ala Asp Val Glu Ser Lys Ser Gly Phe Thr Pro Leu His
   225 230 235 240
   Ile Ala Ala His Tyr Gly Asn Ile Asn Val Ala Thr Leu Leu Leu Asn
   245 250 255
   Arg Ala Ala Ala Val Asp Phe Thr Ala Arg Asn Asp Ile Thr Pro Leu
   260 265 270
   His Val Ala Ser Lys Arg Gly Asn Ala Asn Met Val Lys Leu Leu Leu
   275 280 285
   Asp Arg Gly Ala Lys Ile Asp Ala Lys Thr Arg Asp Gly Leu Thr Pro
   290 295 300
   Leu His Cys Gly Ala Arg Ser Gly His Glu Gln Val Val Glu Met Leu
   305 310 315 320
   Leu Asp Arg Ala Ala Pro Ile Leu Ser Lys Thr Lys Asn Gly Leu Ser
   325 330 335
   Pro Leu His Met Ala Thr Gln Gly Asp His Leu Asn Cys Val Gln Leu
   340 345 350
   Leu Leu Gln His Asn Val Pro Val Asp Asp Val Thr Asn Asp Tyr Leu
   355 360 365
   Thr Ala Leu His Val Ala Ala His Cys Gly His Tyr Lys Val Ala Lys
   370 375 380
   Val Leu Leu Asp Lys Lys Ala Asn Pro Asn Ala Lys Ala Leu Asn Gly
   385 390 395 400
   Phe Thr Pro Leu His Ile Ala Cys Lys Lys Asn Arg Ile Lys Val Met
   405 410 415
   Glu Leu Leu Leu Lys His Gly Ala Ser Ile Gln Ala Val Thr Glu Ser
   420 425 430
   Gly Leu Thr Pro Ile His Val Ala Ala Phe Met Gly His Val Asn Ile
   435 440 445
   Val Ser Gln Leu Met His His Gly Ala Ser Pro Asn Thr Thr Asn Val
   450 455 460
   Arg Gly Glu Thr Ala Leu His Met Ala Ala Arg Ser Gly Gln Ala Glu
   465 470 475 480
   Val Val Arg Tyr Leu Val Gln Asp Gly Ala Gln Val Glu Ala Lys Ala
   485 490 495
   Lys Asp Asp Gln Thr Pro Leu His Ile Ser Ala Arg Leu Gly Lys Ala
   500 505 510
   Asp Ile Val Gln Gln Leu Leu Gln Gln Gly Ala Ser Pro Asn Ala Ala
   515 520 525
   Thr Thr Ser Gly Tyr Thr Pro Leu His Leu Ser Ala Arg Glu Gly His
   530 535 540
   Glu Asp Val Ala Ala Phe Leu Leu Asp His Gly Ala Ser Leu Ser Ile
   545 550 555 560
   Thr Thr Lys Lys Gly Phe Thr Pro Leu His Val Ala Ala Lys Tyr Gly
   565 570 575
   Lys Leu Glu Val Ala Asn Leu Leu Leu Gln Lys Ser Ala Ser Pro Asp
   580 585 590
   Ala Ala Gly Lys Ser Gly Leu Thr Pro Leu His Val Ala Ala His Tyr
   595 600 605
   Asp Asn Gln Lys Val Ala Leu Leu Leu Leu Asp Gln Gly Ala Ser Pro
   610 615 620
   His Ala Ala Ala Lys Asn Gly Tyr Thr Pro Leu His Ile Ala Ala Lys
   625 630 635 640
   Lys Asn Gln Met Asp Ile Ala Thr Thr Leu Leu Glu Tyr Gly Ala Asp
   645 650 655
   Ala Asn Ala Val Thr Arg Gln Gly Ile Ala Ser Val His Leu Ala Ala
   660 665 670
   Gln Glu Gly His Val Asp Met Val Ser Leu Leu Leu Gly Arg Asn Ala
   675 680 685
   Asn Val Asn Leu Ser Asn Lys Ser Gly Leu Thr Pro Leu His Leu Ala
   690 695 700
   Ala Gln Glu Asp Arg Val Asn Val Ala Glu Val Leu Val Asn Gln Gly
   705 710 715 720
   Ala His Val Asp Ala Gln Thr Lys Met Gly Tyr Thr Pro Leu His Val
   725 730 735
   Gly Cys His Tyr Gly Asn Ile Lys Ile Val Asn Phe Leu Leu Gln His
   740 745 750
   Ser Ala Lys Val Asn Ala Lys Thr Lys Asn Gly Tyr Thr Pro Leu His
   755 760 765
   Gln Ala Ala Gln Gln Gly His Thr His Ile Ile Asn Val Leu Leu Gln
   770 775 780
   Asn Asn Ala Ser Pro Asn Glu Leu Thr Val Asn Gly Asn Thr Ala Leu
   785 790 795 800
   Gly Ile Ala Arg Arg Leu Gly Tyr Ile Ser Val Val Asp Thr Leu Lys
   805 810 815
   Ile Val Thr Glu Glu Thr Met Thr Thr Thr Thr Val Thr Glu Lys His
   820 825 830
   Lys Met Asn Val Pro Glu Thr Met Asn Glu Val Leu Asp Met Ser Asp
   835 840 845
   Asp Glu Val Arg Lys Ala Asn Ala Pro Glu Met Leu Ser Asp Gly Glu
   850 855 860
   Tyr Ile Ser Asp Val Glu Glu Gly Glu Asp Ala Met Thr Gly Asp Thr
   865 870 875 880
   Asp Lys Tyr Leu Gly Pro Gln Asp Leu Lys Glu Leu Gly Asp Asp Ser
   885 890 895
   Leu Pro Ala Glu Gly Tyr Met Gly Phe Ser Leu Gly Ala Arg Ser Ala
   900 905 910
   Ser Leu Arg Ser Phe Ser Ser Asp Arg Ser Tyr Thr Leu Asn Arg Ser
   915 920 925
   Ser Tyr Ala Arg Asp Ser Met Met Ile Glu Glu Leu Leu Val Pro Ser
   930 935 940
   Lys Glu Gln His Leu Thr Phe Thr Arg Glu Phe Asp Ser Asp Ser Leu
   945 950 955 960
   Arg His Tyr Ser Trp Ala Ala Asp Thr Leu Asp Asn Val Asn Leu Val
   965 970 975
   Ser Ser Pro Ile His Ser Gly Phe Leu Val Ser Phe Met Val Asp Ala
   980 985 990
   Arg Gly Gly Ser Met Arg Gly Ser Arg His His Gly Met Arg Ile Ile
   995 1000 1005
   Ile Pro Pro Arg Lys Cys Thr Ala Pro Thr Arg Ile Thr Cys Arg
   1010 1015 1020
   Leu Val Lys Arg His Lys Leu Ala Asn Pro Pro Pro Met Val Glu
   1025 1030 1035
   Gly Glu Gly Leu Ala Ser Arg Leu Val Glu Met Gly Pro Ala Gly
   1040 1045 1050
   Ala Gln Phe Leu Gly Pro Val Ile Val Glu Ile Pro His Phe Gly
   1055 1060 1065
   Ser Met Arg Gly Lys Glu Arg Glu Leu Ile Val Leu Arg Ser Glu
   1070 1075 1080
   Asn Gly Glu Thr Trp Lys Glu His Gln Phe Asp Ser Lys Asn Glu
   1085 1090 1095
   Asp Leu Thr Glu Leu Leu Asn Gly Met Asp Glu Glu Leu Asp Ser
   1100 1105 1110
   Pro Glu Glu Leu Gly Lys Lys Arg Ile Cys Arg Ile Ile Thr Lys
   1115 1120 1125
   Asp Phe Pro Gln Tyr Phe Ala Val Val Ser Arg Ile Lys Gln Glu
   1130 1135 1140
   Ser Asn Gln Ile Gly Pro Glu Gly Gly Ile Leu Ser Ser Thr Thr
   1145 1150 1155
   Val Pro Leu Val Gln Ala Ser Phe Pro Glu Gly Ala Leu Thr Lys
   1160 1165 1170
   Arg Ile Arg Val Gly Leu Gln Ala Gln Pro Val Pro Asp Glu Ile
   1175 1180 1185
   Val Lys Lys Ile Leu Gly Asn Lys Ala Thr Phe Ser Pro Ile Val
   1190 1195 1200
   Thr Val Glu Pro Arg Arg Arg Lys Phe His Lys Pro Ile Thr Met
   1205 1210 1215
   Thr Ile Pro Val Pro Pro Pro Ser Gly Glu Gly Val Ser Asn Gly
   1220 1225 1230
   Tyr Lys Gly Asp Thr Thr Pro Asn Leu Arg Leu Leu Cys Ser Ile
   1235 1240 1245
   Thr Gly Gly Thr Ser Pro Ala Gln Trp Glu Asp Ile Thr Gly Thr
   1250 1255 1260
   Thr Pro Leu Thr Phe Ile Lys Asp Cys Val Ser Phe Thr Thr Asn
   1265 1270 1275
   Val Ser Ala Arg Phe Trp Leu Ala Asp Cys His Gln Val Leu Glu
   1280 1285 1290
   Thr Val Gly Leu Ala Thr Gln Leu Tyr Arg Glu Leu Ile Cys Val
   1295 1300 1305
   Pro Tyr Met Ala Lys Phe Val Val Phe Ala Lys Met Asn Asp Pro
   1310 1315 1320
   Val Glu Ser Ser Leu Arg Cys Phe Cys Met Thr Asp Asp Lys Val
   1325 1330 1335
   Asp Lys Thr Leu Glu Gln Gln Glu Asn Phe Glu Glu Val Ala Arg
   1340 1345 1350
   Ser Lys Asp Ile Glu Val Leu Glu Gly Lys Pro Ile Tyr Val Asp
   1355 1360 1365
   Cys Tyr Gly Asn Leu Ala Pro Leu Thr Lys Gly Gly Gln Gln Leu
   1370 1375 1380
   Val Phe Asn Phe Tyr Ser Phe Lys Glu Asn Arg Leu Pro Phe Ser
   1385 1390 1395
   Ile Lys Ile Arg Asp Thr Ser Gln Glu Pro Cys Gly Arg Leu Ser
   1400 1405 1410
   Phe Leu Lys Glu Pro Lys Thr Thr Lys Gly Leu Pro Gln Thr Ala
   1415 1420 1425
   Val Cys Asn Leu Asn Ile Thr Leu Pro Ala His Lys Lys Glu Thr
   1430 1435 1440
   Glu Ser Asp Gln Asp Asp Glu Ile Glu Lys Thr Asp Arg Arg Gln
   1445 1450 1455
   Ser Phe Ala Ser Leu Ala Leu Arg Lys Arg Tyr Ser Tyr Leu Thr
   1460 1465 1470
   Glu Pro Gly Met Ile Glu Arg Ser Thr Gly Ala Thr Arg Ser Leu
   1475 1480 1485
   Pro Thr Thr Tyr Ser Tyr Lys Pro Phe Phe Ser Thr Arg Pro Tyr
   1490 1495 1500
   Gln Ser Trp Thr Thr Ala Pro Ile Thr Val Pro Gly Pro Ala Lys
   1505 1510 1515
   Ser Gly Phe Thr Ser Leu Ser Ser Ser Ser Ser Asn Thr Pro Ser
   1520 1525 1530
   Ala Ser Pro Leu Lys Ser Ile Trp Ser Val Ser Thr Pro Ser Pro
   1535 1540 1545
   Ile Lys Ser Thr Leu Gly Ala Ser Thr Thr Ser Ser Val Lys Ser
   1550 1555 1560
   Ile Ser Asp Val Ala Ser Pro Ile Arg Ser Phe Arg Thr Met Ser
   1565 1570 1575
   Ser Pro Ile Lys Thr Val Val Ser Gln Ser Pro Tyr Asn Ile Gln
   1580 1585 1590
   Val Ser Ser Gly Thr Leu Ala Arg Ala Pro Ala Val Thr Glu Ala
   1595 1600 1605
   Thr Pro  Leu Lys Gly Leu Ala  Ser Asn Ser Thr Phe  Ser Ser Arg
       1610                 1615                 1620
   Thr Ser  Pro Val Thr Thr Ala  Gly Ser Leu Leu Glu  Arg Ser Ser
       1625                 1630                 1635
   Ile Thr  Met Thr Pro Pro Ala  Ser Pro Lys Ser Asn  Ile Asn Met
       1640                 1645                 1650
   Tyr Ser  Ser Ser Leu Pro Phe  Lys Ser Ile Ile Thr  Ser Ala Ala
       1655                 1660                 1665
   Pro Leu  Ile Ser Ser Pro Leu  Lys Ser Val Val Ser  Pro Val Lys
       1670                 1675                 1680
   Ser Ala  Val Asp Val Ile Ser  Ser Ala Lys Ile Thr  Met Ala Ser
       1685                 1690                 1695
   Ser Leu  Ser Ser Pro Val Lys  Gln Met Pro Gly His  Ala Glu Val
       1700                 1705                 1710
   Ala Leu  Val Asn Gly Ser Ile  Ser Pro Leu Lys Tyr  Pro Ser Ser
       1715                 1720                 1725
   Ser Thr  Leu Ile Asn Gly Cys  Lys Ala Thr Ala Thr  Leu Gln Glu
       1730                 1735                 1740
   Lys Ile  Ser Ser Ala Thr Asn  Ser Val Ser Ser Val  Val Ser Ala
       1745                 1750                 1755
   Ala Thr  Asp Thr Val Glu Lys  Val Phe Ser Thr Thr  Thr Ala Met
       1760                 1765                 1770
   Pro Phe  Ser Pro Leu Arg Ser  Tyr Val Ser Ala Ala  Pro Ser Ala
       1775                 1780                 1785
   Phe Gln  Ser Leu Arg Thr Pro  Ser Ala Ser Ala Leu  Tyr Thr Ser
       1790                 1795                 1800
   Leu Gly  Ser Ser Ile Ser Ala  Thr Thr Ser Ser Val  Thr Ser Ser
       1805                 1810                 1815
   Ile Ile  Thr Val Pro Val Tyr  Ser Val Val Asn Val  Leu Pro Glu
       1820                 1825                 1830
   Pro Ala  Leu Lys Lys Leu Pro  Asp Ser Asn Ser Phe  Thr Lys Ser
       1835                 1840                 1845
   Ala Ala Ala Leu Leu Ser Pro Ile Lys Thr Leu Thr Thr Glu Thr
   1850 1855 1860
   His Pro Gln Pro His Phe Ser Arg Thr Ser Ser Pro Val Lys Ser
   1865 1870 1875
   Ser Leu Phe Leu Ala Pro Ser Ala Leu Lys Leu Ser Thr Pro Ser
   1880 1885 1890
   Ser Leu Ser Ser Ser Gln Glu Ile Leu Lys Asp Val Ala Glu Met
   1895 1900 1905
   Lys Glu Asp Leu Met Arg Met Thr Ala Ile Leu Gln Thr Asp Val
   1910 1915 1920
   Pro Glu Glu Lys Pro Phe Gln Pro Glu Leu Pro Lys Glu Gly Arg
   1925 1930 1935
   Ile Asp Asp Glu Glu Pro Phe Lys Ile Val Glu Lys Val Lys Glu
   1940 1945 1950
   Asp Leu Val Lys Val Ser Glu Ile Leu Lys Lys Asp Val Cys Val
   1955 1960 1965
   Asp Asn Lys Gly Ser Pro Lys Ser Pro Lys Ser Asp Lys Gly His
   1970 1975 1980
   Ser Pro Glu Asp Asp Trp Ile Glu Phe Ser Ser Glu Glu Ile Arg
   1985 1990 1995
   Glu Ala Arg Gln Gln Ala Ala Ala Ser Gln Ser Pro Ser Leu Pro
   2000 2005 2010
   Glu Arg Val Gln Val Lys Ala Lys Ala Ala Ser Glu Lys Asp Tyr
   2015 2020 2025
   Asn Leu Thr Lys Val Ile Asp Tyr Leu Thr Asn Asp Ile Gly Ser
   2030 2035 2040
   Ser Ser Leu Thr Asn Leu Lys Tyr Lys Phe Glu Asp Ala Lys Lys
   2045 2050 2055
   Asp Gly Glu Glu Arg Gln Lys Arg Val Leu Lys Pro Ala Ile Ala
   2060 2065 2070
   Leu Gln Glu His Lys Leu Lys Met Pro Pro Ala Ser Met Arg Thr
   2075 2080 2085
   Ser Thr Ser Glu Lys Glu Leu Cys Lys Met Ala Asp Ser Phe Phe
   2090 2095 2100
   Gly Thr Asp Thr Ile Leu Glu Ser Pro Asp Asp Phe Ser Gln His
   2105 2110 2115
   Asp Gln Asp Lys Ser Pro Leu Ser Asp Ser Gly Phe Glu Thr Arg
   2120 2125 2130
   Ser Glu Lys Thr Pro Ser Ala Pro Gln Ser Ala Glu Ser Thr Gly
   2135 2140 2145
   Pro Lys Pro Leu Phe His Glu Val Pro Ile Pro Pro Val Ile Thr
   2150 2155 2160
   Glu Thr Arg Thr Glu Val Val His Val Ile Arg Ser Tyr Asp Pro
   2165 2170 2175
   Ser Ala Gly Asp Val Pro Gln Thr Gln Pro Glu Glu Pro Val Ser
   2180 2185 2190
   Pro Lys Pro Ser Pro Thr Phe Met Glu Leu Glu Pro Lys Pro Thr
   2195 2200 2205
   Thr Ser Ser Ile Lys Glu Lys Val Lys Ala Phe Gln Met Lys Ala
   2210 2215 2220
   Ser Ser Glu Glu Asp Asp His Asn Arg Val Leu Ser Lys Gly Met
   2225 2230 2235
   Arg Val Lys Glu Glu Thr His Ile Thr Thr Thr Thr Arg Met Val
   2240 2245 2250
   Tyr His Ser Pro Pro Gly Gly Glu Gly Ala Ser Glu Arg Ile Glu
   2255 2260 2265
   Glu Thr Met Ser Val His Asp Ile Met Lys Ala Phe Gln Ser Gly
   2270 2275 2280
   Arg Asp Pro Ser Lys Glu Leu Ala Gly Leu Phe Glu His Lys Ser
   2285 2290 2295
   Ala Val Ser Pro Asp Val His Lys Ser Ala Ala Glu Thr Ser Ala
   2300 2305 2310
   Gln His Ala Glu Lys Asp Asn Gln Met Lys Pro Lys Leu Glu Arg
   2315 2320 2325
   Ile Ile Glu Val His Ile Glu Lys Gly Asn Gln Ala Glu Pro Thr
   2330 2335 2340
   Glu Val Ile Ile Arg Glu Thr Lys Lys His Pro Glu Lys Glu Met
   2345 2350 2355
   Tyr Val Tyr Gln Lys Asp Leu Ser Arg Gly Asp Ile Asn Leu Lys
   2360 2365 2370
   Asp Phe Leu Pro Glu Lys His Asp Ala Phe Pro Cys Ser Glu Glu
   2375 2380 2385
   Gln Gly Gln Gln Glu Glu Glu Glu Leu Thr Ala Glu Glu Ser Leu
   2390 2395 2400
   Pro Ser Tyr Leu Glu Ser Ser Arg Val Asn Thr Pro Val Ser Gln
   2405 2410 2415
   Glu Glu Asp Ser Arg Pro Ser Ser Ala Gln Leu Ile Ser Asp Asp
   2420 2425 2430
   Ser Tyr Lys Thr Leu Lys Leu Leu Ser Gln His Ser Ile Glu Tyr
   2435 2440 2445
   His Asp Asp Glu Leu Ser Glu Leu Arg Gly Glu Ser Tyr Arg Phe
   2450 2455 2460
   Ala Glu Lys Met Leu Leu Ser Glu Lys Leu Asp Val Ser His Ser
   2465 2470 2475
   Asp Thr Glu Glu Ser Val Thr Asp His Ala Gly Pro Pro Ser Ser
   2480 2485 2490
   Glu Leu Gln Gly Ser Asp Lys Arg Ser Arg Glu Lys Ile Ala Thr
   2495 2500 2505
   Ala Pro Lys Lys Glu Ile Leu Ser Lys Ile Tyr Lys Asp Val Ser
   2510 2515 2520
   Glu Asn Gly Val Gly Lys Val Ser Lys Asp Glu His Phe Asp Lys
   2525 2530 2535
   Val Thr Val Leu His Tyr Ser Gly Asn Val Ser Ser Pro Lys His
   2540 2545 2550
   Ala Met Trp Met Arg Phe Thr Glu Asp Arg Leu Asp Arg Gly Arg
   2555 2560 2565
   Glu Lys Leu Ile Tyr Glu Asp Arg Val Asp Arg Thr Val Lys Glu
   2570 2575 2580
   Ala Glu Glu Lys Leu Thr Glu Val Ser Gln Phe Phe Arg Asp Lys
   2585 2590 2595
   Thr Glu Lys Leu Asn Asp Glu Leu Gln Ser Pro Glu Lys Lys Ala
   2600 2605 2610
   Arg Pro Lys Asn Gly Lys Glu Tyr Ser Ser Gln Ser Pro Thr Ser
   2615 2620 2625
   Ser Ser Pro Glu Lys Val Leu Leu Thr Glu Leu Leu Ala Ser Asn
   2630 2635 2640
   Asp Glu Trp Val Lys Ala Arg Gln His Gly Pro Asp Gly Gln Gly
   2645 2650 2655
   Phe Pro Lys Ala Glu Glu Lys Ala Pro Ser Leu Pro Ser Ser Pro
   2660 2665 2670
   Glu Lys Met Val Leu Ser Gln Gln Thr Glu Asp Ser Lys Ser Thr
   2675 2680 2685
   Val Glu Ala Lys Gly Ser Ile Ser Gln Ser Lys Ala Pro Asp Gly
   2690 2695 2700
   Pro Gln Ser Gly Phe Gln Leu Lys Gln Ser Lys Leu Ser Ser Ile
   2705 2710 2715
   Arg Leu Lys Phe Glu Gln Gly Thr His Ala Lys Ser Lys Asp Met
   2720 2725 2730
   Ser Gln Glu Asp Arg Lys Ser Asp Gly Gln Ser Arg Ile Pro Val
   2735 2740 2745
   Lys Lys Ile Gln Glu Ser Lys Leu Pro Val Tyr Gln Val Phe Ala
   2750 2755 2760
   Arg Glu Lys Gln Gln Lys Ala Ile Asp Leu Pro Asp Glu Ser Val
   2765 2770 2775
   Ser Val Gln Lys Asp Phe Met Val Leu Lys Thr Lys Asp Glu His
   2780 2785 2790
   Ala Gln Ser Asn Glu Ile Val Val Asn Asp Ser Gly Ser Asp Asn
   2795 2800 2805
   Val Lys Lys Gln Arg Thr Glu Met Ser Ser Lys Ala Met Pro Asp
   2810 2815 2820
   Ser Phe Ser Glu Gln Gln Ala Lys Asp Leu Ala Cys His Ile Thr
   2825 2830 2835
   Ser Asp Leu Ala Thr Arg Gly Pro Trp Asp Lys Lys Val Phe Arg
   2840 2845 2850
   Thr Trp Glu Ser Ser Gly Ala Thr Asn Asn Lys Ser Gln Lys Glu
   2855 2860 2865
   Lys Leu Ser His Val Leu Val His Asp Val Arg Glu Asn His Ile
   2870 2875 2880
   Gly His Pro Glu Ser Lys Ser Val Asp Gln Lys Asn Glu Phe Met
   2885 2890 2895
   Ser Val Thr Glu Arg Glu Arg Lys Leu Leu Thr Asn Gly Ser Leu
   2900 2905 2910
   Ser Glu Ile Lys Glu Met Thr Val Lys Ser Pro Ser Lys Lys Val
   2915 2920 2925
   Leu Tyr Arg Glu Tyr Val Val Lys Glu Gly Asp His Pro Gly Gly
   2930 2935 2940
   Leu Leu Asp Gln Pro Ser Arg Arg Ser Glu Ser Ser Ala Val Ser
   2945 2950 2955
   His Ile Pro Val Arg Val Ala Asp Glu Arg Arg Met Leu Ser Ser
   2960 2965 2970
   Asn Ile Pro Asp Gly Phe Cys Glu Gln Ser Ala Phe Pro Lys His
   2975 2980 2985
   Glu Leu Ser Gln Lys Leu Ser Gln Ser Ser Met Ser Lys Glu Thr
   2990 2995 3000
   Val Glu Thr Gln His Phe Asn Ser Ile Glu Asp Glu Lys Val Thr
   3005 3010 3015
   Tyr Ser Glu Ile Ser Lys Val Ser Lys His Gln Ser Tyr Val Gly
   3020 3025 3030
   Leu Cys Pro Pro Leu Glu Glu Thr Glu Thr Ser Pro Thr Lys Ser
   3035 3040 3045
   Pro Asp Ser Leu Glu Phe Ser Pro Gly Lys Glu Ser Pro Ser Ser
   3050 3055 3060
   Asp Val Phe Asp His Ser Pro Ile Asp Gly Leu Glu Lys Leu Ala
   3065 3070 3075
   Pro Leu Ala Gln Thr Glu Gly Gly Lys Glu Ile Lys Thr Leu Pro
   3080 3085 3090
   Val Tyr Val Ser Phe Val Gln Val Gly Lys Gln Tyr Glu Lys Glu
   3095 3100 3105
   Ile Gln Gln Gly Gly Val Lys Lys Ile Ile Ser Gln Glu Cys Lys
   3110 3115 3120
   Thr Val Gln Glu Thr Arg Gly Thr Phe Tyr Thr Thr Arg Gln Gln
   3125 3130 3135
   Lys Gln Pro Pro Ser Pro Gln Gly Ser Pro Glu Asp Asp Thr Leu
   3140 3145 3150
   Glu Gln Val Ser Phe Leu Asp Ser Ser Gly Lys Ser Pro Leu Thr
   3155 3160 3165
   Pro Glu Thr Pro Ser Ser Glu Glu Val Ser Tyr Glu Phe Thr Ser
   3170 3175 3180
   Lys Thr Pro Asp Ser Leu Ile Ala Tyr Ile Pro Gly Lys Pro Ser
   3185 3190 3195
   Pro Ile Pro Glu Val Ser Glu Glu Ser Glu Glu Glu Glu Gln Ala
   3200 3205 3210
   Lys Ser Thr Ser Leu Lys Gln Thr Thr Val Glu Glu Thr Ala Val
   3215 3220 3225
   Glu Arg Glu Met Pro Asn Asp Val Ser Lys Asp Ser Asn Gln Arg
   3230 3235 3240
   Pro Lys Asn Asn Arg Val Ala Tyr Ile Glu Phe Pro Pro Pro Pro
   3245 3250 3255
   Pro Leu Asp Ala Asp Gln Ile Glu Ser Asp Lys Lys His His Tyr
   3260 3265 3270
   Leu Pro Glu Lys Glu Val Asp Met Ile Glu Val Asn Leu Gln Asp
   3275 3280 3285
   Glu His Asp Lys Tyr Gln Leu Ala Glu Pro Val Ile Arg Val Gln
   3290 3295 3300
   Pro Pro Ser Pro Val Pro Pro Gly Ala Asp Val Ser Asp Ser Ser
   3305 3310 3315
   Asp Asp Glu Ser Ile Tyr Gln Pro Val Pro Val Lys Lys Tyr Thr
   3320 3325 3330
   Phe Lys Leu Lys Glu Val Asp Asp Glu Gln Lys Glu Lys Pro Lys
   3335 3340 3345
   Ala Ser Ala Glu Lys Ala Ser Asn Gln Lys Glu Leu Glu Ser Asn
   3350 3355 3360
   Gly Ser Gly Lys Asp Asn Glu Phe Gly Leu Gly Leu Asp Ser Pro
   3365 3370 3375
   Gln Asn Glu Ile Ala Gln Asn Gly Asn Asn Asp Gln Ser Ile Thr
   3380 3385 3390
   Glu Cys Ser Ile Ala Thr Thr Ala Glu Phe Ser His Asp Thr Asp
   3395 3400 3405
   Ala Thr Glu Ile Asp Ser Leu Asp Gly Tyr Asp Leu Gln Asp Glu
   3410 3415 3420
   Asp Asp Gly Leu Thr Glu Ser Asp Ser Lys Leu Pro Ile Gln Ala
   3425 3430 3435
   Met Glu Ile Lys Lys Asp Ile Trp Asn Thr Glu Gly Ile Leu Lys
   3440 3445 3450
   Pro Ala Asp Arg Ser Phe Ser Gln Ser Lys Leu Glu Val Ile Glu
   3455 3460 3465
   Glu Glu Gly Lys Val Gly Pro Asp Glu Asp Lys Pro Pro Ser Lys
   3470 3475 3480
   Ser Ser Ser Ser Glu Lys Thr Pro Asp Lys Thr Asp Gln Lys Ser
   3485 3490 3495
   Gly Ala Gln Phe Phe Thr Leu Glu Gly Arg His Pro Asp Arg Ser
   3500 3505 3510
   Val Phe Pro Asp Thr Tyr Phe Ser Tyr Lys Val Asp Glu Glu Phe
   3515 3520 3525
   Ala Thr Pro Phe Lys Thr Val Ala Thr Lys Gly Leu Asp Phe Asp
   3530 3535 3540
   Pro Trp Ser Asn Asn Arg Gly Asp Asp Glu Val Phe Asp Ser Lys
   3545 3550 3555
   Ser Arg Glu Asp Glu Thr Lys Pro Phe Gly Leu Ala Val Glu Asp
       3560                3565                3570
   Arg Ser Pro Ala Thr Thr Pro Asp Thr Thr Pro Ala Arg Thr Pro
       3575                3580                3585
   Thr Asp Glu Ser Thr Pro Thr Ser Glu Pro Asn Pro Phe Pro Phe
       3590                3595                3600
   His Glu Gly Lys Met Phe Glu Met Thr Arg Ser Gly Ala Ile Asp
       3605                3610                3615
   Met Ser Lys Arg Asp Phe Val Glu Glu Arg Leu Gln Phe Phe Gln
       3620                3625                3630
   Ile Gly Glu His Thr Ser Glu Gly Lys Ser Gly Asp Gln Gly Glu
       3635                3640                3645
   Gly Asp Lys Ser Met Val Thr Ala Thr Pro Gln Pro Gln Ser Gly
       3650                3655                3660
   Asp Thr Thr Val Glu Thr Asn Leu Glu Arg Asn Val Glu Thr Pro
       3665                3670                3675
   Thr Val Glu Pro Asn Pro Ser Ile Pro Thr Ser Gly Glu Cys Gln
       3680                3685                3690
   Glu Gly Thr Ser Ser Ser Gly Ser Leu Glu Lys Ser Ala Ala Ala
       3695                3700                3705
   Thr Asn Thr Ser Lys Val Asp Pro Lys Leu Arg Thr Pro Ile Lys
       3710                3715                3720
   Met Gly Ile Ser Ala Ser Thr Met Thr Met Lys Lys Glu Gly Pro
       3725                3730                3735
   Gly Glu Ile Thr Asp Lys Ile Glu Ala Val Met Thr Ser Cys Gln
       3740                3745                3750
   Gly Leu Glu Asn Glu Thr Ile Thr Met Ile Ser Asn Thr Ala Asn
       3755                3760                3765
   Ser Gln Met Gly Val Arg Pro His Glu Lys His Asp Phe Gln Lys
       3770                3775                3780
   Asp Asn Phe Asn Asn Asn Asn Asn Leu Asp Ser Ser Thr Ile Gln
       3785                3790                3795
   Thr Asp Asn Ile Met Ser Asn Ile Val Leu Thr Glu His Ser Ala
   3800 3805 3810
   Pro Thr Cys Thr Thr Glu Lys Asp Asn Pro Val Lys Val Ser Ser
   3815 3820 3825
   Gly Lys Lys Thr Gly Val Leu Gln Gly His Cys Val Arg Asp Lys
   3830 3835 3840
   Gln Lys Val Leu Gly Glu Gln Gln Lys Thr Lys Glu Leu Ile Gly
   3845 3850 3855
   Ile Arg Gln Lys Ser Lys Leu Pro Ile Lys Ala Thr Ser Pro Lys
   3860 3865 3870
   Asp Thr Phe Pro Pro Asn His Met Ser Asn Thr Lys Ala Ser Lys
   3875 3880 3885
   Met Lys Gln Val Ser Gln Ser Glu Lys Thr Lys Ala Leu Thr Thr
   3890 3895 3900
   Ser Ser Cys Val Asp Val Lys Ser Arg Ile Pro Val Lys Asn Thr
   3905 3910 3915
   His Arg Asp Asn Ile Ile Ala Val Arg Lys Ala Cys Ala Thr Gln
   3920 3925 3930
   Lys Gln Gly Gln Pro Glu Lys Gly Lys Ala Lys Gln Leu Pro Ser
   3935 3940 3945
   Lys Leu Pro Val Lys Val Arg Ser Thr Cys Val Thr Thr Thr Thr
   3950 3955 3960
   Thr Thr Ala Thr Thr Thr Thr Thr Thr Thr Thr Thr Thr Thr Thr
   3965 3970 3975
   Ser Cys Thr Val Lys Val Arg Lys Ser Gln Leu Lys Glu Val Cys
   3980 3985 3990
   Lys His Ser Ile Glu Tyr Phe Lys Gly Ile Ser Gly Glu Thr Leu
   3995 4000 4005
   Lys Leu Val Asp Arg Leu Ser Glu Glu Glu Lys Lys Met Gln Ser
   4010 4015 4020
   Glu Leu Ser Asp Glu Glu Glu Ser Thr Ser Arg Asn Thr Ser Leu
   4025 4030 4035
   Ser Glu Thr Ser Arg Gly Gly Gln Pro Ser Val Thr Thr Lys Ser
   4040 4045 4050
   Ala Arg Asp Lys Lys Thr Glu Ala Ala Pro Leu Lys Ser Lys Ser
   4055 4060 4065
   Glu Lys Ala Gly Ser Glu Lys Arg Ser Ser Arg Arg Thr Gly Pro
   4070 4075 4080
   Gln Ser Pro Cys Glu Arg Thr Asp Ile Arg Met Ala Ile Val Ala
   4085 4090 4095
   Asp His Leu Gly Leu Ser Trp Thr Glu Leu Ala Arg Glu Leu Asn
   4100 4105 4110
   Phe Ser Val Asp Glu Ile Asn Gln Ile Arg Val Glu Asn Pro Asn
   4115 4120 4125
   Ser Leu Ile Ser Gln Ser Phe Met Leu Leu Lys Lys Trp Val Thr
   4130 4135 4140
   Arg Asp Gly Lys Asn Ala Thr Thr Asp Ala Leu Thr Ser Val Leu
   4145 4150 4155
   Thr Lys Ile Asn Arg Ile Asp Ile Val Thr Leu Leu Glu Gly Pro
   4160 4165 4170
   Ile Phe Asp Tyr Gly Asn Ile Ser Gly Thr Arg Ser Phe Ala Asp
   4175 4180 4185
   Glu Asn Asn Val Phe His Asp Pro Val Asp Gly Trp Gln Asn Glu
   4190 4195 4200
   Thr Ser Ser Gly Asn Leu Glu Ser Cys Ala Gln Ala Arg Arg Val
   4205 4210 4215
   Thr Gly Gly Leu Leu Asp Arg Leu Asp Asp Ser Pro Asp Gln Cys
   4220 4225 4230
   Arg Asp Ser Ile Thr Ser Tyr Leu Lys Gly Glu Ala Gly Lys Phe
   4235 4240 4245
   Glu Ala Asn Gly Ser His Thr Glu Ile Thr Pro Glu Ala Lys Thr
   4250 4255 4260
   Lys Ser Tyr Phe Pro Glu Ser Gln Asn Asp Val Gly Lys Gln Ser
   4265 4270 4275
   Thr Lys Glu Thr Leu Lys Pro Lys Ile His Gly Ser Gly His Val
   4280 4285 4290
   Glu Glu Pro Ala Ser Pro Leu Ala Ala Tyr Gln Lys Ser Leu Glu
   4295 4300 4305
   Glu Thr Ser Lys Leu Ile Ile Glu Glu Thr Lys Pro Cys Val Pro
   4310 4315 4320
   Val Ser Met Lys Lys Met Ser Arg Thr Ser Pro Ala Asp Gly Lys
   4325 4330 4335
   Pro Arg Leu Ser Leu His Glu Glu Glu Gly Ser Ser Gly Ser Glu
   4340 4345 4350
   Gln Lys Gln Gly Glu Gly Phe Lys Val Lys Thr Lys Lys Glu Ile
   4355 4360 4365
   Arg His Val Glu Lys Lys Ser His Ser
   4370 4375
<210> 2
   <211> 1001
   <212> PRT
   <213> Homo sapiens
<300>
   <301> Kapfhamer D, Miller DE, Lambert S, Bennett V, Glover TW, Burmeister M.
   <302> Chromosomal localization of the ankyrinG gene (ANK3/Ank3) to human 10q21 and mouse 10.
   <303> Genomics
   <304> 27
   <305> 1
   <306> 189-91
   <307> 1995-05-01
   <308> PMID/7665168
   <309> 1995-10-12
   <313> (1)..(1001)
<300>
   <301> Lee MT, Chen CH, Lee CS, Chen CC, Chong MY, Ouyang WC, Chiu NY, Chuo LJ, Chen CY, Tan HK, Lane HY, Chang TJ, Lin CH, Jou SH, Hou YM, Feng J, Lai TJ, Tung CL, Chen TJ, Chang CJ, Lung FW, Chen CK, Shiah IS, Liu CY, Teng PR, Chen KH, Shen LJ, Cheng CS, Chang TP, Li CF, Chou CH, Chen CY, Wang KH, Fann CS, Wu JY, Chen YT, Cheng AT.
   <302> Genome-wide association study of bipolar I disorder in the Han Chinese population.
   <303> Molecular Psychiatry
   <304> 00
   <306> 00
   <307> 2010-04-13
   <308> PMID/20386566
   <309> 2010-04-13
<300>
   <301> Williams HJ, Craddock N, Russo G, Hamshere ML, Moskvina V, Dwyer S, Smith RL, Green E, Grozeva D, Holmans P, Owen MJ, O'Donovan MC
   <302> Most genome-wide significant susceptibility loci for schizophrenia and bipolar disorder reported to date cross-traditional diagnostic boundaries.
   <303> Human Molecular Genetics
   <304> 20
   <305> 2
   <306> 387-391
   <307> 2011-01-15
   <308> PMID/21037240
   <309> 2010-12-23
   <313> (1)..(1001)
<300>
   <308> UniProtKB/BlAQT2
   <309> 2008-04-08
   <313> (1)..(1001)
<400> 2
   Met Ala Leu Pro Gln Ser Glu Asp Ala Met Thr Gly Asp Thr Asp Lys
   1               5                   10                  15
   Tyr Leu Gly Pro Gln Asp Leu Lys Glu Leu Gly Asp Asp Ser Leu Pro
               20                  25                  30
   Ala Glu Gly Tyr Met Gly Phe Ser Leu Gly Ala Arg Ser Ala Ser Leu
           35                  40                  45
   Arg Ser Phe Ser Ser Asp Arg Ser Tyr Thr Leu Asn Arg Ser Ser Tyr
       50                  55                  60
   Ala Arg Asp Ser Met Met Ile Glu Glu Leu Leu Val Pro Ser Lys Glu
   65                  70                  75                  80
   Gln His Leu Thr Phe Thr Arg Glu Phe Asp Ser Asp Ser Leu Arg His
                   85                  90                  95
   Tyr Ser Trp Ala Ala Asp Thr Leu Asp Asn Val Asn Leu Val Ser Ser
               100                 105                 110
   Pro Ile His Ser Gly Phe Leu Val Ser Phe Met Val Asp Ala Arg Gly
           115                 120                 125
   Gly Ser Met Arg Gly Ser Arg His His Gly Met Arg Ile Ile Ile Pro
       130                 135                 140
   Pro Arg Lys Cys Thr Ala Pro Thr Arg Ile Thr Cys Arg Leu Val Lys
   145                 150                 155                 160
   Arg His Lys Leu Ala Asn Pro Pro Pro Met Val Glu Gly Glu Gly Leu
                   165                 170                 175
   Ala Ser Arg Leu Val Glu Met Gly Pro Ala Gly Ala Gln Phe Leu Gly
               180                 185                 190
   Pro Val Ile Val Glu Ile Pro His Phe Gly Ser Met Arg Gly Lys Glu
   195 200 205
   Arg Glu Leu Ile Val Leu Arg Ser Glu Asn Gly Glu Thr Trp Lys Glu
   210 215 220
   His Gln Phe Asp Ser Lys Asn Glu Asp Leu Thr Glu Leu Leu Asn Gly
   225 230 235 240
   Met Asp Glu Glu Leu Asp Ser Pro Glu Glu Leu Gly Lys Lys Arg Ile
   245 250 255
   Cys Arg Ile Ile Thr Lys Asp Phe Pro Gln Tyr Phe Ala Val Val Ser
   260 265 270
   Arg Ile Lys Gln Glu Ser Asn Gln Ile Gly Pro Glu Gly Gly Ile Leu
   275 280 285
   Ser Ser Thr Thr Val Pro Leu Val Gln Ala Ser Phe Pro Glu Gly Ala
   290 295 300
   Leu Thr Lys Arg Ile Arg Val Gly Leu Gln Ala Gln Pro Val Pro Asp
   305 310 315 320
   Glu Ile Val Lys Lys Ile Leu Gly Asn Lys Ala Thr Phe Ser Pro Ile
   325 330 335
   Val Thr Val Glu Pro Arg Arg Arg Lys Phe His Lys Pro Ile Thr Met
   340 345 350
   Thr Ile Pro Val Pro Pro Pro Ser Gly Glu Gly Val Ser Asn Gly Tyr
   355 360 365
   Lys Gly Asp Thr Thr Pro Asn Leu Arg Leu Leu Cys Ser Ile Thr Gly
   370 375 380
   Gly Thr Ser Pro Ala Gln Trp Glu Asp Ile Thr Gly Thr Thr Pro Leu
   385 390 395 400
   Thr Phe Ile Lys Asp Cys Val Ser Phe Thr Thr Asn Val Ser Ala Arg
   405 410 415
   Phe Trp Leu Ala Asp Cys His Gln Val Leu Glu Thr Val Gly Leu Ala
   420 425 430
   Thr Gln Leu Tyr Arg Glu Leu Ile Cys Val Pro Tyr Met Ala Lys Phe
   435 440 445
   Val Val Phe Ala Lys Met Asn Asp Pro Val Glu Ser Ser Leu Arg Cys
   450 455 460
   Phe Cys Met Thr Asp Asp Lys Val Asp Lys Thr Leu Glu Gln Gln Glu
   465 470 475 480
   Asn Phe Glu Glu Val Ala Arg Ser Lys Asp Ile Glu Val Leu Glu Gly
   485 490 495
   Lys Pro Ile Tyr Val Asp Cys Tyr Gly Asn Leu Ala Pro Leu Thr Lys
   500 505 510
   Gly Gly Gln Gln Leu Val Phe Asn Phe Tyr Ser Phe Lys Glu Asn Arg
   515 520 525
   Leu Pro Phe Ser Ile Lys Ile Arg Asp Thr Ser Gln Glu Pro Cys Gly
   530 535 540
   Arg Leu Ser Phe Leu Lys Glu Pro Lys Thr Thr Lys Gly Leu Pro Gln
   545 550 555 560
   Thr Ala Val Cys Asn Leu Asn Ile Thr Leu Pro Ala His Lys Lys Ile
   565 570 575
   Glu Lys Thr Asp Arg Arg Gln Ser Phe Ala Ser Leu Ala Leu Arg Lys
   580 585 590
   Arg Tyr Ser Tyr Leu Thr Glu Pro Gly Met Ser Pro Gln Ser Pro Cys
   595 600 605
   Glu Arg Thr Asp Ile Arg Met Ala Ile Val Ala Asp His Leu Gly Leu
   610 615 620
   Ser Trp Thr Glu Leu Ala Arg Glu Leu Asn Phe Ser Val Asp Glu Ile
   625 630 635 640
   Asn Gln Ile Arg Val Glu Asn Pro Asn Ser Leu Ile Ser Gln Ser Phe
   645 650 655
   Met Leu Leu Lys Lys Trp Val Thr Arg Asp Gly Lys Asn Ala Thr Thr
   660 665 670
   Asp Ala Leu Thr Ser Val Leu Thr Lys Ile Asn Arg Ile Asp Ile Val
   675 680 685
   Thr Leu Leu Glu Gly Pro Ile Phe Asp Tyr Gly Asn Ile Ser Gly Thr
   690 695 700
   Arg Ser Phe Ala Asp Glu Asn Asn Val Phe His Asp Pro Val Asp Gly
   705 710 715 720
   Tyr Pro Ser Leu Gln Val Glu Leu Glu Thr Pro Thr Gly Leu His Tyr
   725 730 735
   Thr Pro Pro Thr Pro Phe Gln Gln Asp Asp Tyr Phe Ser Asp Ile Ser
   740 745 750
   Ser Ile Glu Ser Pro Leu Arg Thr Pro Ser Arg Leu Ser Asp Gly Leu
   755 760 765
   Val Pro Ser Gln Gly Asn Ile Glu His Ser Ala Asp Gly Pro Pro Val
   770 775 780
   Val Thr Ala Glu Asp Ala Ser Leu Glu Asp Ser Lys Leu Glu Asp Ser
   785 790 795 800
   Val Pro Leu Thr Glu Met Pro Glu Ala Val Asp Val Asp Glu Ser Gln
   805 810 815
   Leu Glu Asn Val Cys Leu Ser Trp Gln Asn Glu Thr Ser Ser Gly Asn
   820 825 830
   Leu Glu Ser Cys Ala Gln Ala Arg Arg Val Thr Gly Gly Leu Leu Asp
   835 840 845
   Arg Leu Asp Asp Ser Pro Asp Gln Cys Arg Asp Ser Ile Thr Ser Tyr
   850 855 860
   Leu Lys Gly Glu Ala Gly Lys Phe Glu Ala Asn Gly Ser His Thr Glu
   865 870 875 880
   Ile Thr Pro Glu Ala Lys Thr Lys Ser Tyr Phe Pro Glu Ser Gln Asn
   885 890 895
   Asp Val Gly Lys Gln Ser Thr Lys Glu Thr Leu Lys Pro Lys Ile His
   900 905 910
   Gly Ser Gly His Val Glu Glu Pro Ala Ser Pro Leu Ala Ala Tyr Gln
   915 920 925
   Lys Ser Leu Glu Glu Thr Ser Lys Leu Ile Ile Glu Glu Thr Lys Pro
   930 935 940
   Cys Val Pro Val Ser Met Lys Lys Met Ser Arg Thr Ser Pro Ala Asp
   945 950 955 960
   Gly Lys Pro Arg Leu Ser Leu His Glu Glu Glu Gly Ser Ser Gly Ser
   965 970 975
   Glu Gln Lys Gln Gly Glu Gly Phe Lys Val Lys Thr Lys Lys Glu Ile
               980                 985                 990
   Arg His Val Glu Lys Lys Ser His  Ser
           995                 1000

## Description

### Field of the invention

The present invention generally relates to the technical field of medicine, in particular to the field of neurodegenerative, neurological and protein misfolding disorders such as amyloidosis. More specifically, the invention relates to a surprising role of ankyrin G (ankG) in ß-amyloid (Aß) clearance and in amyloid precursor protein (APP) metabolism.

### Background of the invention

The neuronal protein ankyrin G (ankG/ANKG) is a member of the highly conserved family of ankyrin proteins which are involved in anchoring and transporting membrane and cytoplasmic proteins to the actin-spectrin cytoskeleton (1). Ankyrin G (also known as ankyrin 3, ANK3, and node of Ranvier (ankyrinG)) is part of the actin-spectrin cytoskeleton of neurons, forming an intracellular scaffolding protein which directs and anchors proteins to specialised membrane domains such as the axon initial segment (AIS) of hippocampal neurons (2), indicating a role of ankG in intracellular transport. For example, neural cell adhesion molecules including L1CAMs are localised to the neuronal cell membrane by ankG (3, 4). Targeted disruption of ankG expression in the mouse cerebellum resulted in progressive ataxia and abolished localisation of voltage-gated Na⁺ channels and NrCAMs molecules to the AIS and also progressive Purkinje neuron degeneration is known to occur (4). Recently, ankG was found to be of essential importance in coordinating the trafficking and the ordered distribution of molecules which are present at the AIS of hippocampal neurons (5). In addition, different classes of transmembrane channels require ankG to be properly located. For instance, ankG is required for transport of cyclic nucleotide-gated channels to the plasma membrane of rod photoreceptor sensory cilia outer segments (6). The regulated targeting and concentration of these proteins not only help the adhesion between cells but also cell signalling. Furthermore, ankG expression determines the concentration and accumulation of proteins targeted to the AIS which is exemplified by the fact that any AIS protein not bound to ankG and hence the spectrin cytoskeleton are being endocytosed and are not expressed at the cell membrane (7). This ability of ankG to regulate protein densities at the cell membrane is of utmost importance for proper neuronal cell functioning. It can thus be understood that a (pathological) over-expression of ankG could lead to accumulation of membrane proteins which insertion is mediated and regulated by ankG. This is an intriguing hypothesis, especially when looked at from an aberrant protein processing and transporting point of view in neurodegenerative disorders including Alzheimer's disease (AD). Interestingly, scaffolding proteins like ankG have recently been identified as important key players in regulating signalling pathways which control the immune response (8). Besides its capability of transporting and anchoring transmembrane proteins, ankG is involved in the formation of specialised membrane micro-domains (9). This puts ankG in a highly dynamic and essential position in the organisation of the functionality of the neuronal membrane. Transport of ankG to the AIS is thought to be mediated by the regulation of the activity of nuclear factor κB (NFκB) and not by the membrane proteins it binds to (10). The phosphorylated, that is, inactive form of IκB-alpha (IκBα), one of the inhibitors of NFκB, has been discovered to be enriched at the AIS and its activation results in the failure of ankG to be targeted to the AIS, leading to accumulation of ankG in the neuronal cytosol (11). Such disturbances in the NFκB pathway are known to occur in neurodegenerative disorders including Alzheimer's disease (AD) (12). Cytoskeletal disturbances, impaired intraneuronal transport and changes in neuronal membrane dynamics are known factors in AD (13). Interestingly, a possible role for the cytoskeletal protein ankG in AD pathology was suggested by genetic studies showing that the gene encoding for ankG is located on chromosome 10 within a genetic interval linked to late-onset AD (14). Additionally, ankG mRNA expression was found to be about two-fold higher in AD patients (15). As a further potential link to neurological disorders, the *ANKG* gene locus has been found as one of significant bipolar disorder loci tested in schizophrenia (Ferreira et al., Nat. Genet. 40(2008), 1056-1058).

Despite numerous scientific efforts in the last years, no means to cure AD has been found yet but drug treatment methods that delay or ameliorate the symptoms in the early stages only. Since an increasing number of factors has been identified as a possible cause for AD, it is likely that a combination of factors leads to the development of AD in an individual.

As mentioned above, ankG may be one of these responsible factors and have a role in the development of neurodegenerative disorders including Alzheimer's disease. These findings provide thus a basis for a requirement for drugs targeting ankG and its interaction partners as a possible new means to diagnose, prevent, delay and ameliorate the course or to cure said neurodegenerative disorders. These requirements are solved by the embodiments characterized in the claims and described further below.

### Summary of the invention

The present invention is defined by the claims.

Generally, the present invention relates to neuronal protein ankyrin G (ankG/ANKG) both as a drug and diagnostic means as well as drug and diagnostic target especially in the field of neurological degenerative disorders.

The present invention is based inter alia on the surprising finding of ankG to be both abnormally re-distributed and overexpressed within the neuronal soma and to be present extracellularly in AD brain, localised within ß-amyloid plaques. Without intending to be bound by theory, it is hypothesized that this pathological extracellular presence of ankG could lead to an immune response against ankG in AD. Indeed, anti-ankG antibodies were found in AD sera. Surprisingly, endogenously-produced ankG antibodies following active vaccination of APP-transgenic mice with ankG significantly reduced ß-Amyloid plaque pathology. In addition, it was found that anti-ankG antibodies were protective against Aß-induced dendritic spine loss. Obtained data establishes also a role for ankG in APP processing and AD pathology. Altogether, the findings of the present invention support a neuroprotective effect of the endogenous anti-ankG response in AD

Hence, by establishing a role of ankG in APP processing the present invention provides a new insight into the the role of ankG in AD pathology and establishes ankG as a target, drug, diagnostic agent and particularly as a vaccine in the treatment and diagnosis of the pathogenesis of Alzheimer's disease (AD).

### Brief descriptions of the drawings:

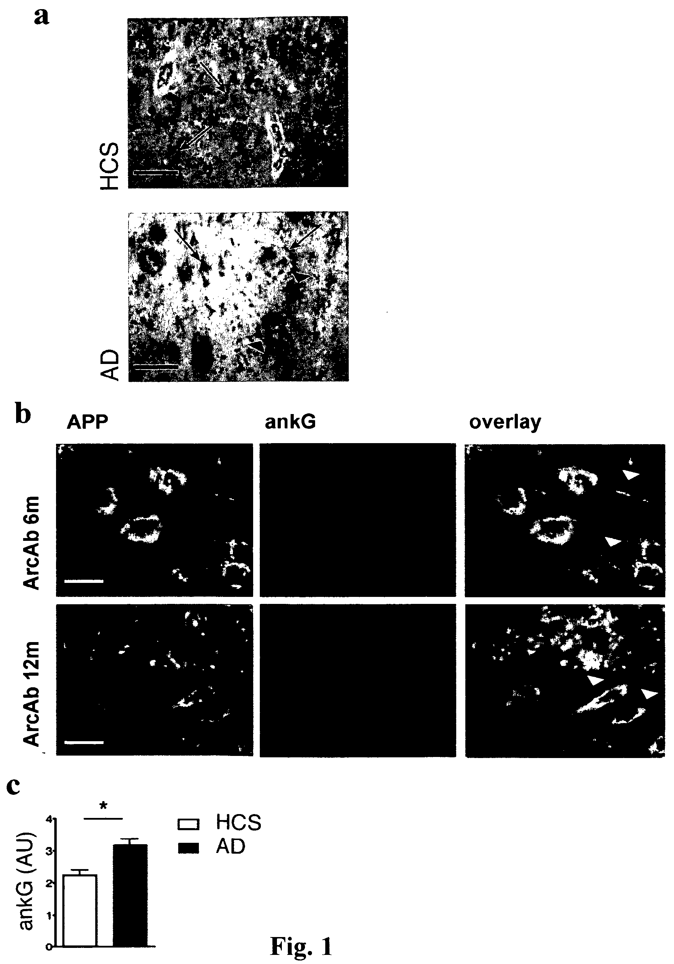

**Fig. 1****: AnkG is present in β-amyloid plaques**
   **(a)** AnkG was present within β-amyloid plaques in the hippocampi of AD patients. Immunohistochemical analysis of paraffin sections from the hippocampi of AD patients was performed using anti-mouse antibody recognizing ankG (red) and anti-human antibody recognizing Aβ (brown). Note the expression of ankG in neurons in both healthy control subjects (HCS) and AD samples (arrow). AnkG-positive structures resembling dystrophic neurites were found in plaques (arrowhead). Scale bar = 50 µm. **(b)** AnkG was present in neuritic plaques in brains from arcAbeta mice. Immunohistochemical staining on 6 and 12 months old arcAbeta mice brains was performed using monoclonal antibodies recognizing ankG (red) and polyclonal antibodies recognizing the C-terminal portion of APP (green). Note the presence of both ankG and APP along the axonal initial segment (AIS) of cortical neurons in the young mouse cortex (arrowhead). In the 12 months old arcAbeta mouse cortex ankG-positive structures were present in the β-amyloid plaques (arrowhead) as seen in the human β-amyloid plaques (d). AnkG immunoreactivity was also observed within dystrophic neurons together with APP. Scale bar = 20 µm. **(c)** Bar graph quantifying ankG immunoreactivity observed in the frontal cortex of AD brains as compared to HCS using tissue micro array analysis. Higher protein levels of ankG were seen in AD versus HCS frontal cortex. Student's t test, p = 0.02 (n = 12).

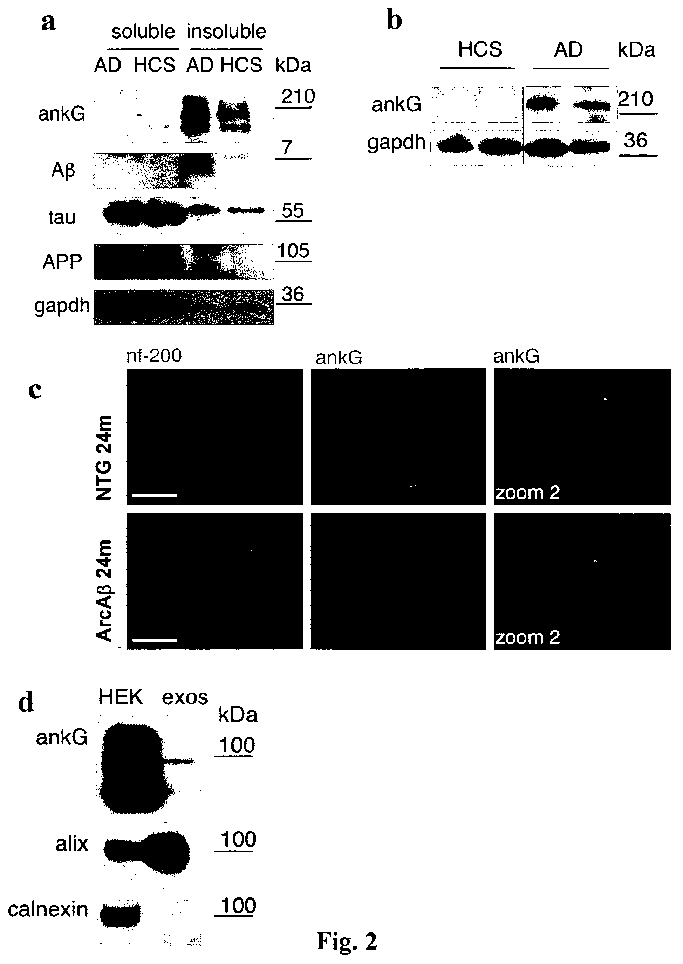

**Fig. 2****: Expression of ankG is deregulated in AD brains and in arcAbeta mouse brains**
   **(a)** WB analysis of SDS soluble and insoluble fractions isolated from AD and HCS hippocampi for ankG, Aβ, tau and APP. AnkG redistributed in the same fraction as Aβ in AD human hippocampus. Note the increased presence of ankG, Aβ and tau in the SDS insoluble fraction from AD as compared to HCS. GAPDH staining indicates that the same amount of total protein was loaded in the AD and HCS lanes for each fraction. **(b)** WB analysis of SDS insoluble fractions from AD versus HCS frontal cortex. Note the increased expression of ankG in the SDS insoluble fraction of the AD affected samples compared to HCS. GAPDH was used as a loading control. **(c)** Immunofluorescence localization of ankG in hippocampal sections of arcAbeta mice and their non-transgenic (NTG) littermates at 24 months of age. Note the redistribution of ankG in the arcAbeta hippocampus when compared to their non-transgenic littermates. A magnified view of the ankG immunofluorescence is presented in the third row to depict the distribution of ankG in the CA3 region of the hippocampus. The hippocampal distribution of neurofilament (nf-200) between arcAbeta mice and their non-transgenic littermates was similar. Scale bar = 300 µm. **(d)** WB analysis of exosomal fractions obtained from HEK 293 cells shows the presence of ankG in exosomes together with the exosomal marker alix. Note the total absence of calnexin from exosomal fraction showing the purity of the preparation.
**Fig**. **3****: AnkG can behave as an antigen triggering the production of specific antibodies in AD patients**
   **(a)** Representative WBs shown evaluating serum immunoreactivity against ankG. The immunoreactive band (≈190 kDa) against ankG shown in AD samples was absent in the HCS. **(b)** Bar graph depicting serum immunoreactivity against ankG evaluated by WBs. Bar graph shows that 8 out of 14 sera samples from AD patients and only 2 out of the 14 sera samples from HCS tested similarly were immunopositive for ankG. Chi-square test, p = 0.018. (c) Analysis of serum IgG immunoreactivity in AD patients by protein expression arrays. Incubation of the protein expression array with sera from a representative AD patient. A 3 cm x 3 cm section of the 24 cm x 24 cm array is shown. IgG immunoreactivity of the AD sera to the expression clone for ankG (spotted in duplicate) is indicated by arrows.

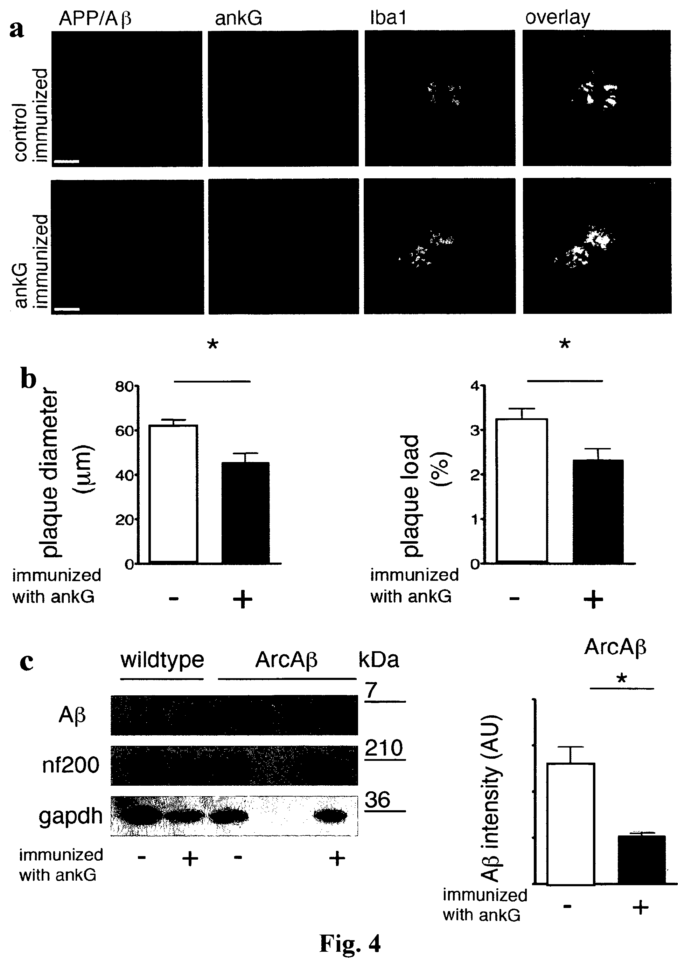

**Fig. 4****: AnkG immunization of arcAbeta mice results in the production of antibodies against ankG and induces microglia-mediated clearance of β-amyloid**
   **(a)** A representative β-Amyloid plaque stained with antibodies recognizing APP/Aβ (blue), ankG (red) and ionized calcium binding adaptor molecule 1 (Iba-1 / green) is shown for immunized and non-immunized arcAbeta mice. Iba-1 is a specific marker of macrophages/microglial cells in the brain (Ito et al., Brain Res. Mol. Brain Res. 57 (1998), 1-9; Thomas WE, Brain Res Brain Res Rev. 17 (1992), 61-74). Note the reduction in plaque size in ankG-immunized as compared to control-immunized arcAbeta mice. An overlapping staining was observed for APP/Aβ and ankG and APP/Aβ, ankG and Iba-1. Note the higher expression / number of Iba-1 immunoreactive cells within the plaque after ankG immunization. Scale bar = 20 µm.
   **(b)** Quantitative image analysis of β-amyloid plaques in the cortex of arcAbeta mice immunized with ankG as compared to control-immunized mice. The diameter (p = 0.026) of the β-amyloid plaques and the plaque load (p = 0.055) were significantly reduced in the cortex of ankG-immunized arcAbeta mice. Mean values ± SEM (Student's t-test, n = 3). Immunization experiments were repeated three times with 3 mice in each group for each experiment. **(c)** Representative WBs showing the levels of Aβ, nf-200 and GAPDH in the SDS insoluble brain fractions from ankG-immunized and control-immunized arcAbeta mice and their non-transgenic littermates. Aβ levels were decreased whereas neurofilament and GAPDH were not affected upon immunization of arcAbeta mice with ankG. Bar graphs show densitometric quantification of the WBs for Aβ levels in ankG and control-immunized arcAbeta mice. Aβ intensities were normalized to GAPDH (n = 3, Mann-Whitney test, p = 0.005).

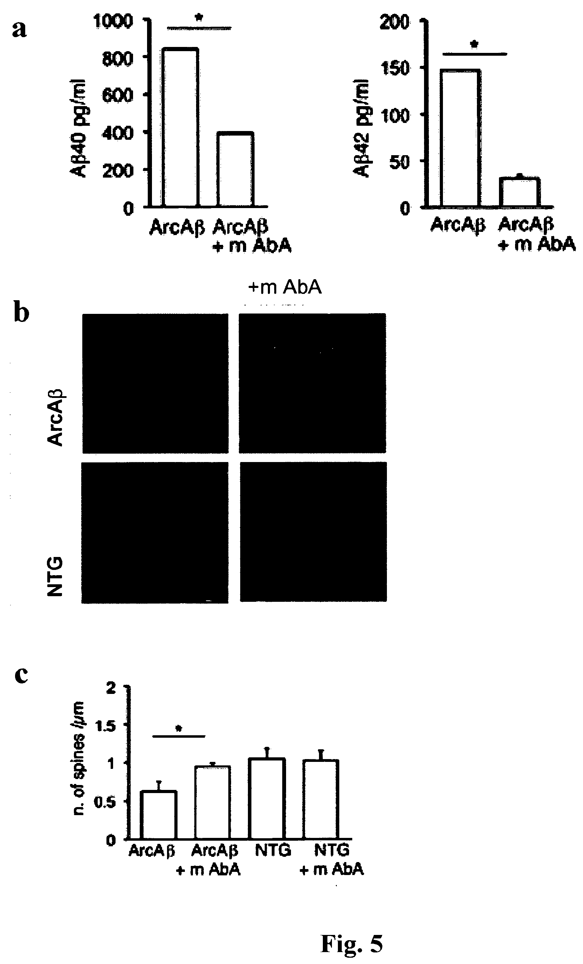

**Fig. 5****: Monoclonal antibodies against ankG lower Aβ levels and Aβ-induced spine loss in ex vivo hippocampal slice cultures from arcAbeta mice**
   **(a)** Quantitative bar graphs representing mean values of the amount of Aβ40 and Aβ42 peptide in medium of arcAbeta hippocampal slice cultures after treatment with a monoclonal antibody against ankG (mAbA) for 1 week. ELISA analysis showed a decrease in Aβ40 and Aβ42 peptides as compared to control immunization (n = 6, Student's t test, p = 0.0002 for Aβ42, p = 0.0003 for Aβ40). **(b)** Representative high-resolution confocal images of dendritic segments from CA3 apical dendrites of EGFP-expressing neurons from hippocampal slice cultures of arcAbeta mice and their non-transgenic littermates. (Stratum radiatum, scale bar: 5µm). Spine density is strongly reduced in arcAbeta neurons. Note the protective effect of the anti-ankG antibody (mAbA) as compared to untreated arcAbeta cultures. Anti-ankG antibody treatment does not affect spine density in non-transgenic controls. **(c)** Quantification of spine density in EGFP-expressing neurons from hippocampal slice cultures of arcAbeta mice and their non-transgenic littermates. Spines were analyzed in CA1 and CA3 apical dendrites and the results were pooled. Values are shown as average (n=10, Student's t test, *p*=0.00001).
**Fig**. **6****: AnkG interacts directly with APP in the brain**
   **(a)** AnkG and APP immunoprecipitates from human frontal cortex were immunoblotted with specific antibodies against ankG, APP and ankB. Immunoprecipitates with non-immune IgG served as a negative control. Note that APP co-immunoprecipitated with ankG and *vice versa.* Human brain homogenate (C) was used as positive control. **(b)** AnkG immunoprecipitates were obtained from arcAbeta (TG), non-transgenic (NTG) littermates and APP deficient (-/-) mouse brains. Note that APP, but not ankB, co-immunoprecipitated with ankG. Mouse brain homogenate (C) was used as positive control. **(c)** Primary rat hippocampal cultures were stained with antibodies against the C-terminal portion of APP (green) and with antibodies against ankG (red). Note the presence of both ankG and APP in the AIS. Scale bar = 10 µm (upper panels) and Scale bar = 30 µm (lower panels). **(d)** ELISA binding curve shows the direct interaction of purified ankG to APP at its intracellular domain (AICD50) coated on ELISA plates (5 µg/ml). AnkG binding to BSA-coated wells (2 mg/ml) served as negative control. Mean values (OD₄₅₀) ± SEM are shown (n=6). **(e)** AnkG and APP immunoprecipitates from human AD hippocampus were immunoblotted with specific antibodies against APP and Caspr and L1. Immunoprecipitates with non-immune IgG served as a negative control. As already shown in **(a)** APP co-immunoprecipitated with ankG. Note the absence of Caspr and L1 from the immunoprecipitates. AD hippocampus homogenate (+) was used as positive control.
**Fig**. **7****: Silencing of ankG results in altered trafficking of APP**
   **(a)** Surface biotinylation to analyze the effects of ankG siRNA silencing on the trafficking of APP to the cell surface in SHY-5Y neuroblastoma cells. WB analysis showed a reduction in cell surface biotinylated APP after ankG silencing as compared to non-silenced or ankB-silenced cells. The presence of the cell adhesion molecule caspr at the cell surface was not affected by silencing (n=3). **(b)** Immunocytochemistry of APP-citrine overexpressing HEK293 cells showed an intracellular accumulation of citrine-APP within the cells and almost a lack of APP at the cell membrane after silencing of ankG. Silencing of ankB did not affect surface localisation of APP although some intracellular accumulation could be seen. Scale bar = 20 µm. **(c)** WB analysis of cell lysates of ankG silenced HEK293. Reduced amounts of the α-CTF were detected in lysates from cells treated with ankG siRNA as compared to cells treated with ankB siRNA and non-silenced cells. Cells were pre-treated with the γ-secretase inhibitor DAPT to obtain detectable levels of α-CTF. Actin was used as a loading control. (Student's t test, p = 0.009, n=6). **(d)** Quantitative bar graphs showing the Aβ40 reduction as observed by ELISA in the medium of HeLa cells expressing the Swedish APP mutation after ankG silencing as compared to control silenced cells (Student's t test, p = 0.004, n=9).
**Fig**. **8****: (a)** Increase in antibody titres against ankG in sera from arcAbeta mice after monthly subcutaneous immunizations with recombinant ankG protein as determined by ELISA, and plotted at different time points. Note that high serum titres of anti-ankG antibodies were detectable after the third month of immunization. **(b)** Mouse antibodies after ankG immunization were found within β-amyloid plaques. Scale bar = 20 µm. **(c)** Quantitative bar graphs representing mean values of the amount of Aβ40 and Aβ42 peptide in the SDS insoluble fractions. ELISA analysis showed a decrease in Aβ40 and Aβ42 peptides in arcAbeta mice after ankG immunization as compared to control immunization (n = 3, Mann-Whitney test, p = 0.03 for Aβ42, p = 0.04 for Aβ40). **(d)** ELISA assays to quantify the amount of antibodies against Aβ40 and Aβ42 peptides in arcAbeta mice after ankG immunization as compared to arcAbeta control-immunized. Note that there was no increase in anti-Aβ40 and Aβ42 antibodies after ankG immunization. **(e, f)** Quantitative bar graphs representing mean values of the amount of Aβ42 peptide in the SDS soluble fractions. ELISA analysis showed an increase in Aβ42 **(e)** (Student's t test, p = 0.02, n=24) but not in Aβ40 (**f**) (Student's t test, p >0.05, n=24). **(g)** Quantitative bar graphs representing mean values of the amount of Aβ42 peptide in the formic acid extracted SDS insoluble fractions from APPSwe mice. ELISA analysis showed a decrease in Aβ42 peptides after ankG immunization as compared to controls (n = 4, Student's t test, p = 0.01). **(h)** Quantitative bar graphs representing mean values of the amount of Aβ40 peptide in the formic acid extracted SDS insoluble fractions from APPSwe mice. ELISA analysis showed no differences in Aβ40 peptides after ankG immunization as compared to controls (n = 4, Student's t test, p = 0.8). **(i)** Quantitative bar graphs representing mean values of the amount of Aβ42 peptide in the sera of APPSwe mice immunized with ankG. ELISA analysis showed an increase in Aβ42 peptides after ankG immunization as compared to controls (n = 4, Student's t test, p = 0.04).
**Fig**. **9****: (a)** Quantification **of Iba1** mean intensity per plaque area shows an increased staining within plaques of arcAbeta mice after ankG immunization as compared to control-immunized arcAbeta mice. **(b)** Quantitative bar graphs representing mean values of the amount of Aβ42 peptide in the formic acid extracted SDS insoluble fractions from APPSwe mice. ELISA analysis showed a decrease in Aβ42 peptides after ankG immunization as compared to controls (n = 4, Student's t test, p = 0.007). (c) Quantitative bar graphs representing mean values of the amount of Aβ42 peptide in the sera of APPSwe mice immunized with ankG. ELISA analysis showed an increase in Aβ42 peptides after ankG immunization as compared to controls (n = 4, Student's t test, p = 0.009). **(d)** WB showing a strong immunoreactivity for recombinant ankG of monoclonal antibodies against ankG obtained after ankG active immunization of arcAbeta mice. Note that there is no cross-reactivity with the high homologous recombinant protein ankB.

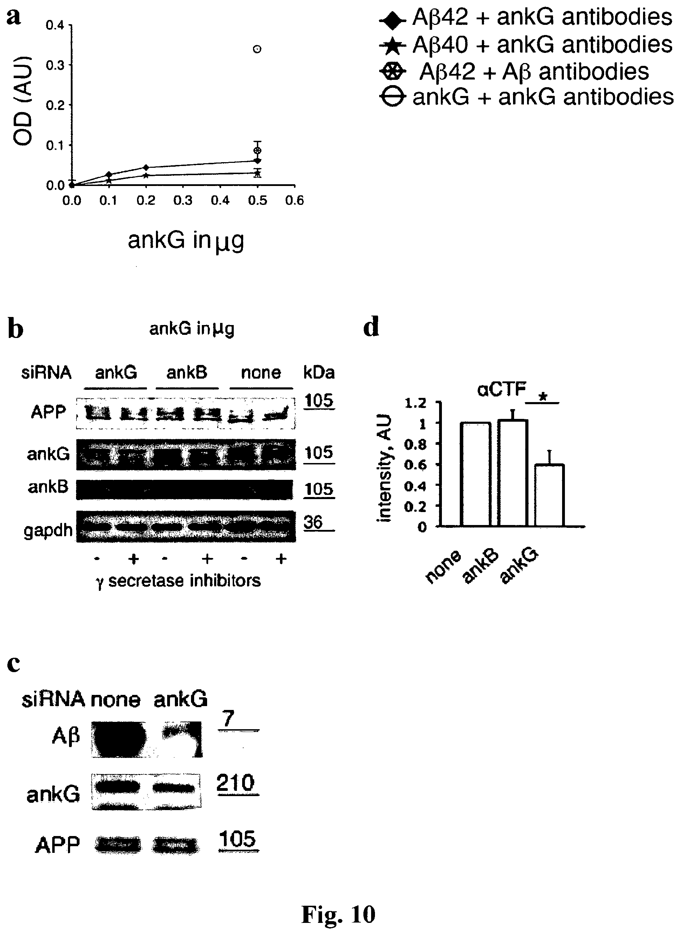

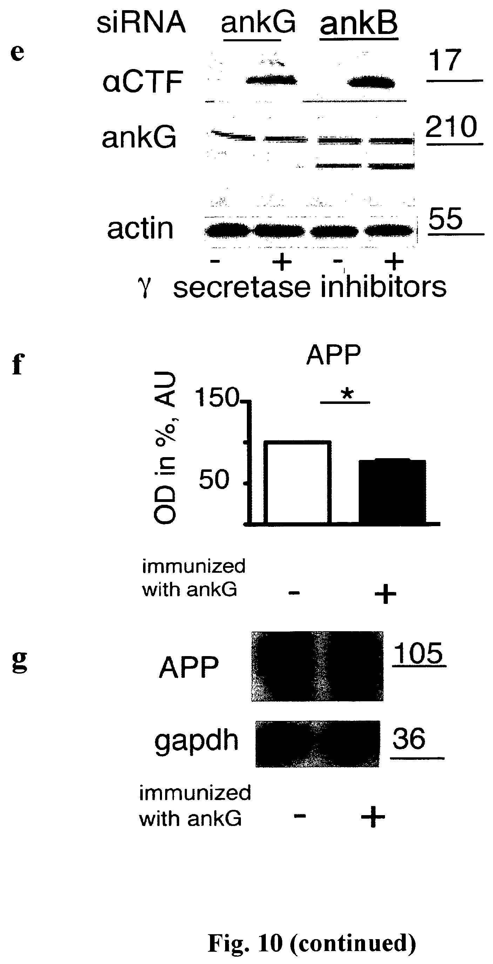

**Fig. 10****: (a)** ELISA binding curves indicate no direct binding between ankG and fibrillar Aβ40 or Aβ42. AnkG and Aβ42 recognized by specific antibodies served as positive controls for the functionality of the assay. Mean values (OD₄₅₀) ± SEM (n = 3) are shown. **(b)** Cell lysates were immunoblotted and analyzed for protein levels of APP, ankG, ankB and GAPDH after being treated with γ secretase inhibitor and ankG siRNA, ankB siRNA or silenced controls. Decreased levels of ankG and ankB corresponded to the ankG and ankB siRNA treatment. Full length APP was not decreased after silencing of ankG or ankB as compared to the silenced controls. GAPDH was used as a loading control (n=6). (c) Cell lysates of overexpressing Swedish APP HEK cells were immunoblotted and analyzed for protein levels of Aβ, APP and ankG after ankG silencing or silenced controls. Decreased levels of Aβ were observed after ankG silencing. Full length APP was not decreased after silencing of ankG (n=3). **(d)** Histogram representing the α-CTF reduction after ankG silencing as compared to cells silenced for ankB and non-silenced cells. **(e)** WB analysis of cell lysates of SH-SY5Y ankG silenced cells. Reduced amounts of the α-CTF were detected in lysates from cells treated with ankG siRNA as compared to cells treated with ankB siRNA. Cells were pre-treated with the γ-secretase inhibitor DAPT to obtain detectable levels of α-CTF. Actin was used as a loading control. (Student's t test, p = 0.02, n=6). **(f)** Histogram representing the APP reduction in SDS soluble fractions after ankG immunization in arcAbeta mice. **(g)** SDS soluble fraction of arcAbeta immunized mice were immunoblotted and analysed for poteins levels of APP. Note the reduced presence of APP after immunization as compared to controls (Student's t test, p = 0.03, n=6).

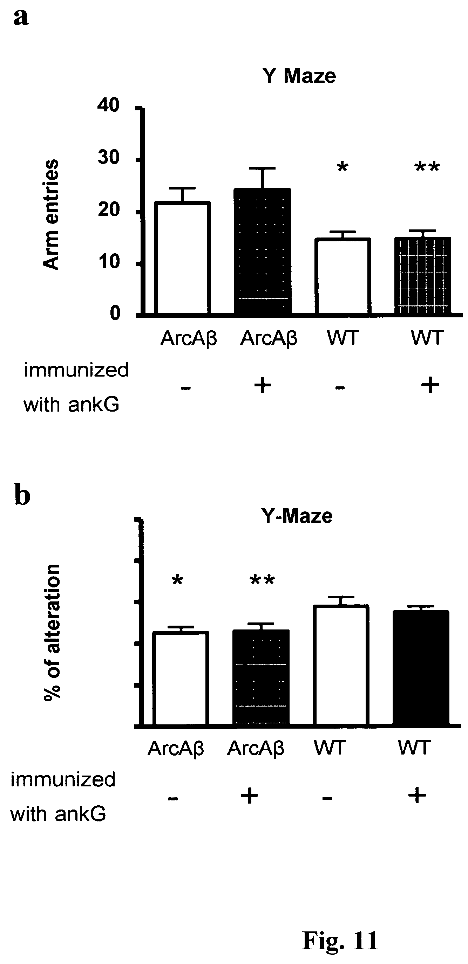

**Fig.11****: AnkG-immunization is not neurotoxic and does not interfere with known physiological functions of ankG in neurotransmission**
   **(a)** Four groups of littermate mice, including non transgenic (n= 23), arcAbeta (n = 23), non transgenic immunized (n = 23) and arcAbeta-immunized (n = 25) mice, were subjected to Y-Maze. Mice were individually placed into a radial symmetric-maze and the total number of arm entries (y axis) was estimated for each group (x axis). Transgenic mice demonstrated significantly higher numbers of arm entries as compared to non-transgenic mice, as already described (17). No significant difference was found between wildtype and wildtype-immunized and transgenic and transgenic immunized. Data are represented as group means ± s.e.m. All *post hoc* statistical comparisons are versus non transgenic mice, *p = 0.05, **p= 0.001. **(b)** Percentage alternation between Y-maze arms (y axis) is represented for each genotype (x axis). No significant difference was found between non transgenic and non transgenic-immunized and transgenic and transgenic immunized (Anova test, P > 0.05). Data are represented as group means s.e.m. All post hoc statistical comparisons are versus non transgenic mice, *p = 0.05, **p= 0.04.

### Definitions

Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2.

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

The term "peptide" is understood to include the terms "polypeptide" and "protein" (which, at times, may be used interchangeably herein) within its meaning. Similarly, fragments of proteins and polypeptides are also contemplated and may be referred to herein as "peptides". Nevertheless, the term "peptide" preferably denotes an amino acid polymer including at least 5 contiguous amino acids, preferably at least 10 contiguous amino acids, more preferably at least 15 contiguous amino acids, still more preferably at least 20 contiguous amino acids, and particularly preferred at least 25 contiguous amino acids. In addition, the peptide in accordance with present invention typically has no more than 100 contiguous amino acids, preferably less than 80 contiguous amino acids and more preferably less than 50 contiguous amino acids.

As used herein, the term "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, "peptides," "dipeptides," "tripeptides, "oligopeptides," "protein," "amino acid chain," or any other term used to refer to a chain or chains of two or more amino acids, are included within the definition of "polypeptide," and the term "polypeptide" may be used instead of, or interchangeably with any of these terms.

The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis.

A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Nevertheless, the term "polypeptide" preferably denotes an amino acid polymer including at least 100 amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded. As used herein, the term glycoprotein refers to a protein coupled to at least one carbohydrate moiety that is attached to the protein via an oxygen-containing or a nitrogen-containing side chain of an amino acid residue, *e.g*., a serine residue or an asparagine residue.

By an "isolated" polypeptide or a fragment, variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for purposed of the invention, as are native or recombinant polypeptides which have been separated, fractionated, or partially or substantially purified by any suitable technique.

"Recombinant peptides, polypeptides or proteins" refer to peptides, polypeptides or proteins produced by recombinant DNA techniques, *i.e.* produced from cells, microbial or mammalian, transformed by an exogenous recombinant DNA expression construct encoding the fusion protein including the desired peptide. Proteins or peptides expressed in most bacterial cultures will typically be free of glycan. Proteins or polypeptides expressed in yeast may have a glycosylation pattern different from that expressed in mammalian cells.

Also included as polypeptides of the present disclosure comprising the invention are fragments, derivatives, analogs or variants of the foregoing polypeptides, and any combination thereof. The terms "fragment," "variant," "derivative" and "analog" include peptides and polypeptides having an amino acid sequence sufficiently similar to the amino acid sequence of the natural peptide. The term "sufficiently similar" means a first amino acid sequence that contains a sufficient or minimum number of identical or equivalent amino acid residues relative to a second amino acid sequence such that the first and second amino acid sequences have a common structural domain and/or common functional activity. For example, amino acid sequences that comprise a common structural domain that is at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100%, identical are defined herein as sufficiently similar. Preferably, variants will be sufficiently similar to the amino acid sequence of the preferred peptides of the present invention, in particular to ankG, APP or fragments, variants, derivatives or analogs of either of them, in particular wherein said fragment derivative or analog is derived from the intracytoplasmatic domain of APP. Such variants generally retain the functional activity of the peptides of the present invention. Variants include peptides that differ in amino acid sequence from the native and wt peptide, respectively, by way of one or more amino acid deletion(s), addition(s), and/or substitution(s). These may be naturally occurring variants as well as artificially designed ones.

"Similarity" between two peptides is determined by comparing the amino acid sequence of one peptide to the sequence of a second peptide. An amino acid of one peptide is similar to the corresponding amino acid of a second peptide if it is identical or a conservative amino acid substitution. Conservative substitutions include those described in Dayhoff, M.O., ed., The Atlas of Protein Sequence and Structure 5, National Biomedical Research Foundation, Washington, D.C. (1978), and in Argos, EMBO J. 8 (1989), 779-785. For example, amino acids belonging to one of the following groups represent conservative changes or substitutions: -Ala, Pro, Gly, Gln, Asn, Ser, Thr; -Cys, Ser, Tyr, Thr; -Val, Ile, Leu, Met, Ala, Phe; -Lys, Arg, His; -Phe, Tyr, Trp, His; and -Asp, Glu.

The terms "antibody" and "immunoglobulin" are used interchangeably herein. An antibody or immunoglobulin is an ankG-binding molecule which comprises at least the variable domain of a heavy chain, and normally comprises at least the variable domains of a heavy chain and a light chain. Basic immunoglobulin structures in vertebrate systems are relatively well understood; see, *e.g*., Harlow and Lane, Antibodies: A Laboratory Manual, (Cold Spring Harbor Laboratory Press, 2nd ed. 1988).

Any antibody or immunoglobulin fragment which contains sufficient structure to specifically bind to ankG is denoted herein interchangeably as a "binding fragment" or an "immunospecific fragment."

Antibodies or antigen-binding fragments, immunospecific fragments, variants, or derivatives thereof include, but are not limited to, polyclonal, monoclonal, multi specific, human, humanized, primatized, murinized or chimeric antibodies, single chain antibodies, epitope-binding fragments, *e.g.,* Fab, Fab' and F(ab')₂, Fd, Fvs, single-chain Fvs (scFv), single-chain antibodies, disulfide-linked Fvs (sdFv), fragments comprising either a V_{L} or V_{H} domain, fragments produced by a Fab expression library, and anti-idiotypic (anti-Id) antibodies (including, *e.g.,* anti-Id antibodies to antibodies disclosed herein). ScFv molecules are known in the art and are described, *e.g.,* in US patent 5,892,019. Immunoglobulin or antibody molecules of the invention can be of any type (*e.g.,* IgG, IgE, IgM, IgD, IgA, and IgY), class *(e.g.,* IgG1, IgG2, IgG3, IgG4, IgA1 and IgA2) or subclass of immunoglobulin molecule.

Antibodies or immunospecific fragments thereof may be from any animal origin including birds and mammals. Preferably, the antibodies are human, murine, donkey, rabbit, goat, guinea pig, camel, Ilama, horse, or chicken antibodies. In another embodiment, the variable region may be condricthoid in origin (*e.g*., from sharks).

As used herein, the term "sample" refers to any biological material obtained from a subject or patient. In one aspect, a sample can comprise blood, cerebrospinal fluid ("CSF"), or urine. In other aspects, a sample can comprise whole blood, plasma, B-cells enriched from blood samples, and cultured cells (*e.g*., B-cells from a subject). A sample can also include a biopsy or tissue sample including neural tissue. In still other aspects, a sample can comprise whole cells and/or a lysate of the cells. Blood samples can be collected by methods known in the art. In one aspect, the pellet can be resuspended by vortexing at 4°C in 200 µl buffer (20 mM Tris, pH. 7.5, 0.5% Nonidet, 1 mM EDTA, 1 mM PMSF, 0.1M NaCl, IX Sigma Protease Inhibitor, and IX Sigma Phosphatase Inhibitors 1 and 2). The suspension can be kept on ice for 20 minutes with intermittent vortexing. After spinning at 15,000 x g for 5 minutes at about 4°C, aliquots of supernatant can be stored at about -70°C.

As used herein, the terms "treat" or "treatment" refer to both therapeutic treatment and prophylactic or preventative measures, wherein the object is to prevent or slow down (lessen) an undesired physiological change or disorder, such as the development of Alzheimer's disease. Beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the manifestation of the condition or disorder is to be prevented.

If not stated otherwise the term "drug," "medicine," or "medicament" are used interchangeably herein and shall include but are not limited to all (A) articles, medicines and preparations for internal or external use, and any substance or mixture of substances intended to be used for diagnosis, cure, mitigation, treatment, or prevention of disease of either man or other animals; and (B) articles, medicines and preparations (other than food) intended to affect the structure or any function of the body of man or other animals; and (C) articles intended for use as a component of any article specified in clause (A) and (B). The term "drug," "medicine," or "medicament" shall include the complete formula of the preparation intended for use in either man or other animals containing one or more "agents," "compounds", "substances" or "(chemical) compositions" as and in some other context also other pharmaceutically inactive excipients as fillers, disintegrants, lubricants, glidants, binders or ensuring easy transport, disintegration, disaggregation, dissolution and biological availability of the "drug," "medicine," or "medicament" at an intended target location within the body of man or other animals, *e.g.,* at the skin, in the stomach or the intestine. The terms "agent," "compound" or "substance" are used interchangeably herein and shall include, in a more particular context, but are not limited to all pharmacologically active agents, *i.e.* agents that induce a desired biological or pharmacological effect or are investigated or tested for the capability of inducing such a possible pharmacological effect by the methods of the present invention.

By "subject" or "individual" or "animal" or "patient" or "mammal," is meant any subject, particularly a mammalian subject, e.g., a human patient, for whom diagnosis, prognosis, prevention, or therapy is desired.

### Detailed description of the present invention

Abnormal extracellular and intracellular deposits including the amyloid-ß peptide (Aß) and tau protein underlie the severe brain pathology in Alzheimer's disease (AD) and can induce an AD-related antigen immune response. The present invention is based on the observation of an abnormal extracellular presence of the neuronal cytoskeletal protein ankyrinG (ankG), present both in ß-amyloid plaques and exosomal vesicles. Also an antibody response against ankG was observed which was higher in AD sera than in healthy control subjects (HCS) sera. Additionally, neuronal ankG expression was higher in AD patients than in age-matched healthy control subjects (HCS). Active immunization of APP-transgenic mice with ankG reduced brain ß-amyloid pathology and antibodies against ankG reduced Aß-induced loss of dendritic spines in *ex vivo* cultures. Furthermore, an interaction between ankG and APP was found. Moreover, after ankG silencing a decreased APP cell-surface trafficking and a lower Aß production could be observed. The findings of the present invention, which are described in detail below and in the Examples, establish a surprising role for ankG in ß-amyloid clearance and in APP metabolism. AnkG immunotherapy provides a novel avenue for Aß-lowering therapy.

International application WO 2005/098433 describes experiments with human tissue sections from normal and Alzheimer disease patient donors as well as from the APP23 mouse model over-expressing human APP with the familial 'Swedish' double mutations suggesting that ankG is expressed in plaque-associated dystrophic neurites. However, though these observations led to the suggestion that during the pathogenesis of AD the neuronal distribution or subcellular localization of ankG is changed, further expanded studies were demanded in order to possibly establish whether the presence of ankG correlates with AD and disease progression. Accordingly, the role of ankG as a putative positive or negative marker of AD was still to be established. In this context, international application WO 2007/140973 describes that another member of the Ankyrin family, Ankyrin 2 was found at a decreased level in AD patients suggesting to reflect cytoskeletal impairment of neuronal cells in AD, which may result in a deficit in axonal transporting and neuritic dystrophy.

In contrast, experiments performed in accordance with the present invention revealed an abnormal extracellular presence of the neuronal cytoskeletal ankG in β-amyloid plaques and exosomal vesicles and a higher neuronal ankG expression in AD patients compared to age-matched healthy control subjects. Moreover, the experiments performed in accordance with the present invention revealed ankG as suitable target in the immunotherapy of amyloidosis and other neurodegenerative diseases which are due to abnormal aggregation of proteins.

Therefore, as a new means in the treatment of neurological disorders, the present invention relates to an agent capable of reducing the level of Ankyrin G (ankG), binding to ankG and/or interfering with the binding of ankG to a target protein in the brain for use in the treatment, amelioration or prevention of a neurological disorder, which agent will be referred to as "agent of the present invention" in further description. Put in other words, the present invention generally relates to ankG and derivatives thereof including any agent capable of interfering with the biological activity of ankG as a medicament useful in the treatment of neurological disorders. Regarding the mode of action, the present invention relates to any agent that could interfere with or prevent the (pathological) extracellular distribution and / or localisation of ankyrin G. In this context, the agent may interfere with ankG in the sense of reducing the level of expression of ankG, preventing ankG from binding to the target protein, for example by sequestering ankG and/or blocking its target protein binding domain, and the like. Likewise, ankG and an agent directly derived thereof such as peptides derived from ankG may compete with the binding of native ankG to its target protein. Thus, any agent which is capable of preventing the interaction of native ankG with its target protein in the brain is included within the definition of term "agent" according to the present invention.

The term "ankG/ANKG" is also used to generally identify other conformers of ankG/ANKG, for example, oligomers or aggregates of ankG/ANKG. The term "ankG/ANKG " is also used to refer collectively to all types and forms of ankG/ANKG/Ank3/ANK3.

Two ankG/ANKG-isoforms are annotated in human. The amino acid sequence of ankG/ANKG isoform-1 of 4377aa can be retrieved from the literature and pertinent databases; see, *e.g.,* Databank UniProtKB/Swiss-Prot: locus Q12955 (ANK3_HUMAN); Kordeli et al., J. Biol. Chem. 270 (1995), 2352-2359.

### Said amino acid sequence of ANKG isoform-1 of 4377aa is:

A further isoform, ankG/ANKG isoform-2 of 1001aa, is annotated in Human with the Databank accession number: B1AQT2 (B1AQT2_HUMAN); Kapfhamer et al., Genomics 27 (1), 189-191 (1995); Lee et al., Mol Psychiatry. 2010 Apr 13. [Epub ahead of print]; Williams et al., Hum. Mol. Genet. 20 (2), 387-391 (2011).

### Said amino acid sequence of ANKG isoform-2 of 1001aa is:

Further Ankyrin G variants and splicing isoforms in human and other organisms such as mouse are known to the person skilled in the art. Their nucleotide and amino acid sequences can be retrived from known databases such as UniProtKB/SwissProt/TrEMBL, GenBank, RefSeq, TPA, PIR, PRF and PDB in which context also the above mentioned synonymes for AnkyrinG may be used. Examples for such sequences are annotated in the mouse with the Databank accession numbers UniProtKB/TrEMBL: Q4U260_MOUSE, Q4U205_MOUSE, Q8VC68_MOUSE, Q8CBN3_MOUSE, Q3TSJ8_MOUSE; GenBank: NP_666117.2, NP_733791.2, NP_733925.2 and further published in Okazaki et al., Nature 420 (2002), 563-573, Carninci and Hayashizaki, Methods Enzymol. 303(1999),19-44, Hoock et al., J. Cell Biol. 136 (1997),1059-1070. Further examples of human Ankyrin G variants are Q5JSX5_HUMAN, Q13484_HUMAN, Q59G01_HUMAN and A8KA62_HUMAN; see also Devarajan et al., J. Cell Biol. 133 (1996), 819-830.

In view of the data presented in the examples of the present invention, the agent of the present invention is preferably used in the treatment, amelioration or prevention of a neurological disorder, wherein the disorder is associated with Alzheimer's disease. Furthermore, the disorder is preferably associated with amyloid β (Aβ) pathology/amyloidosis.

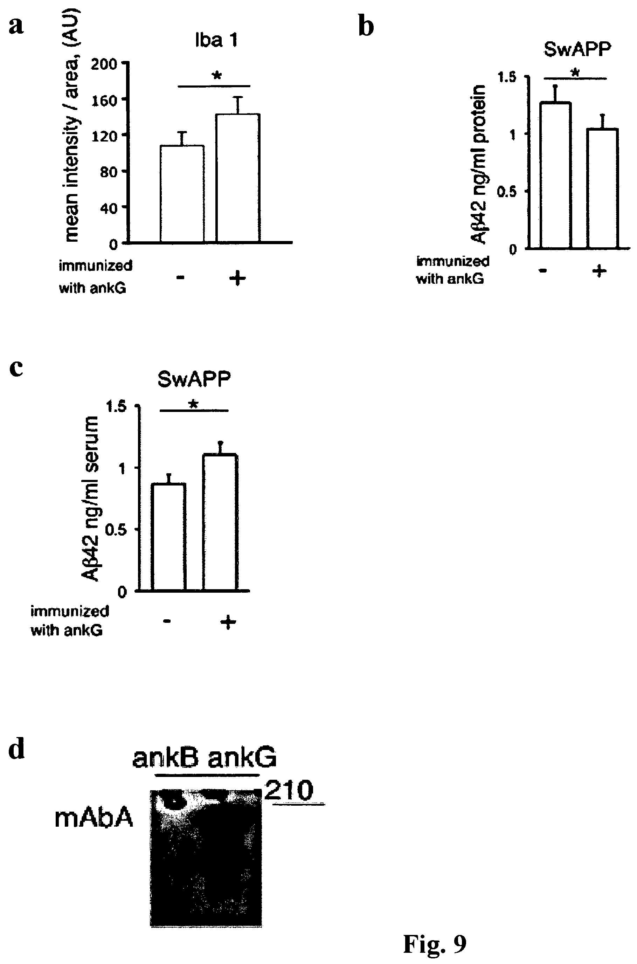

As described in the Examples, active immunization with ankG reduces ß-amyloid pathology, *i.e.* the number and size of ß-amyloid plaques and Aß42 brain levels in Alzheimer's disease model mice; see Example 3 and Figures 4a, 4b and 9b. Therefore, in one preferred embodiment of the present invention said agent is recombinant ankG protein or a fragment, derivative or analog thereof.

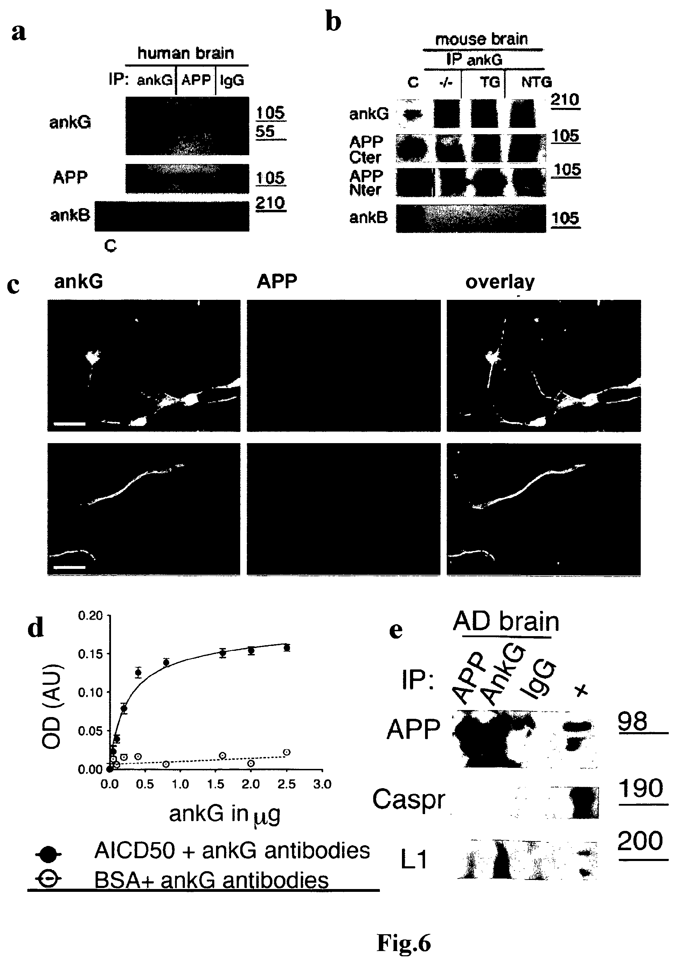

Aß is a hydrophobic peptide and thus the presence of ankG in ß-amyloid plaques could be due to a non-specific interaction with hydrophobic ankG domains. Quite the contrary, as shown in Example 5, no interaction could be found between ankG and Aß. However, APP but not Aß could be shown to interact with ankG in co-immunoprecipitation experiments; see Example 5 and Figure 6a. Thus, according to one embodiment of the present invention the agent of the present invention is capable of interfering with the interaction of ankG and amyloid precursor protein (APP). In a further embodiment of the present invention, the agent of the present invention is capable of detecting and/or binding APP/ankG complexes.

As shown in the examples, antibodies against ankG lower Aß levels and Aß-induced spine loss in hippocampal cultures from said model animals, *i.e.* from arcAbeta mice; see Example 4 and Figures 5 a-c.

As shown in Example 3 of the present invention, active immunization by introduction of peptides or peptide epitopes, *i.e.* vaccination, into a subject can elicit similar effects as passive immunization, *i.e.* provision of antibodies specifically binding said peptides or peptide epitopes; see Example 4. In this context, the person skilled in the art is well aware of the fact that in the manufacture of vaccines, in particular against endogenous peptides or peptide epitopes the purity of the pathological structure used as the antigen is of particular relevance, since any impurity may result in undesired immune reactions, which can manifested themselves in the form of auto-immune-diseases up to septic shock with even lethal consequences. In particular, for preventive medical treatment but also for therapy the safety of a drug is a key criterion, for their development.

Therefore, according to the present invention said agent is formulated as a vaccine which is capable of inducing autoantibodies against ankG.

For use in vaccination said agent has to be formulated, *i.e.* compositions of the active agent, *e.g.* a recombinant peptide or fragment of ankG, with adjuvants has to be produced in such a way as to enhance the bioavailability of the vaccine and the immunological response against it. Almost all adjuvants used today for enhancement of the immune response against antigens are particles or are forming particles together with the antigen, see also "Vaccine Design-the subunit and adjuvant approach" (Ed: Powell & Newman, Plenum Press, 1995) for details. The present invention also relates to a composition for treating a disease, in particular neurodegenerative, neurological or neuropsychiatric disorder comprising the recombinant peptide or fusion protein, as an active agent as described above, and optionally a pharmaceutically acceptable carrier. Similarly, compositions containing peptides, polypeptides or fusion proteins binding, *e.g.,* ankG and pharmaceutically acceptable carriers may be used for diagnosing of said neurodegenerative, neurological or neuropsychiatric disorders.

Pharmaceutically acceptable carriers and administration routes can be taken from corresponding literature known to the person skilled in the art. The pharmaceutical compositions of the present invention can be formulated according to methods well known in the art; see for example Remington: The Science and Practice of Pharmacy (2000) by the University of Sciences in Philadelphia, ISBN 0-683-306472, Vaccine Protocols.2nd Edition by Robinson et al., Humana Press, Totowa, New Jersey, USA, 2003; Banga, Therapeutic Peptides and Proteins: Formulation, Processing, and Delivery Systems. 2nd Edition by Taylor and Francis. (2006), ISBN: 0-8493-1630-8. Examples of suitable pharmaceutical carriers are well known in the art and include phosphate buffered saline solutions, water, emulsions, such as oil/water emulsions, various types of wetting agents, sterile solutions etc. Preferably, the pharmaceutically acceptable carrier is KLH, tetanus toxoid, albumin binding protein, bovine serum albumin, or an adjuvant substance described in Singh et al. , Nat. Biotech. 17 (1999), 1075- 1081 and O'Hagan et al., Nature Reviews, Drug Discovery 2 (9) (2003) , 727- 735, or mixtures thereof. In addition, the vaccine composition may contain aluminium hydroxyde. Compositions comprising such carriers can be formulated by well known conventional methods. These pharmaceutical compositions can be administered to the subject at a suitable dose. Administration of the suitable compositions may be effected by different ways. Examples include administering a composition containing a pharmaceutically acceptable carrier via oral, intranasal, rectal, topical, intraperitoneal, intravenous, intramuscular, subcutaneous, subdermal, transdermal, intrathecal, and intracranial methods. Aerosol formulations such as nasal spray formulations include purified aqueous or other solutions of the active agent with preservative agents and isotonic agents. Such formulations are preferably adjusted to a pH and isotonic state compatible with the nasal mucous membranes.

Formulations for rectal or vaginal administration may be presented as a suppository with a suitable carrier; see also O'Hagan et al., Nature Reviews, Drug Discovery 2(9) (2003), 727-735. Further guidance regarding formulations that are suitable for various types of administration can be found in Remington's Pharmaceutical Sciences, Mace Publishing Company, Philadelphia, PA, 17th ed. (1985) and corresponding updates. For a brief review of methods for drug delivery see Langer, Science 249 (1990), 1527-1533.

The peptides, polypeptides or fusion proteins may be provided by expression in a host cell, for example. To express the peptide, polypeptide or fusion protein in a host cell, the nucleic acid molecule encoding said peptide, polypeptide or fusion protein may be inserted into appropriate expression vector, *i.e.* a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, *and in vivo* genetic recombination. Such techniques are described in Sambrook et al., Molecular Cloning, A Laboratory Manual (1989), and Ausubel et al., Current Protocols in Molecular Biology (1989); see also the literature cited in the Examples section.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (*e.g*., baculovirus); plant cell systems transformed with virus expression vectors (*e.g.*, cauliflower mosaic virus, CaMV; tobacco mosaic virus, TMV) or with bacterial expression vectors (*e.g*., Ti or pBR322 plasmids); or animal cell systems.

To express the peptide, polypeptide or fusion protein (hereinafter referred to as "product") in a host cell, a procedure such as the following can be used. A restriction fragment containing a DNA sequence that encodes said product may be cloned into an appropriate recombinant plasmid containing an origin of replication that functions in the host cell and an appropriate selectable marker. The plasmid may include a promoter for inducible expression of the product (*e.g.,* pTrc (Amann et al, Gene 69 (1988), 301 315) and pET1 Id (Studier et al., Gene Expression Technology: Methods in Enzymology 185, Academic Press, San Diego, Calif. (1990), 60 89). The recombinant plasmid may be introduced into the host cell by, for example, electroporation and cells containing the recombinant plasmid may be identified by selection for the marker on the plasmid. Expression of the product may be induced and detected in the host cell using an assay specific for the product.

A suitable host cell for expression of the product may be any prokaryotic or eukaryotic cell; *e.g.,* bacterial cells such as *E. coli* or *B. subtilis,* insect cells (baculovirus), yeast, or mammalian cells such as Chinese hamster ovary cell (CHO). In some embodiments, the DNA that encodes the product/peptide may be optimized for expression in the host cell. For example, the DNA may include codons for one or more amino acids that are predominant in the host cell relative to other codons for the same amino acid.

Alternatively, the expression of the product may be performed by *in vitro* synthesis of the protein in cell-free extracts which are also particularly suited for the incorporation of modified or unnatural amino acids for functional studies; see also infra. The use of *in vitro* translation systems can have advantages over *in vivo* gene expression when the over-expressed product is toxic to the host cell, when the product is insoluble or forms inclusion bodies, or when the protein undergoes rapid proteolytic degradation by intracellular proteases. The most frequently used cell-free translation systems consist of extracts from rabbit reticulocytes, wheat germ and Escherichia coli. All are prepared as crude extracts containing all the macromolecular components (70S or 80S ribosomes, tRNAs, aminoacyl-tRNA synthetases, initiation, elongation and termination factors, etc.) required for translation of exogenous RNA. To ensure efficient translation, each extract must be supplemented with amino acids, energy sources (ATP, GTP), energy regenerating systems (creatine phosphate and creatine phosphokinase for eukaryotic systems, and phosphoenol pyruvate and pyruvate kinase for the *E. coli* lysate), and other co-factors (Mg²⁺, K⁺, etc.). Appropriate transcription/translation systems are commercially available, for example from Promega Corporation, Roche Diagnostics, and Ambion, *i.e.* Applied Biosystems.

As shown in the examples, the recombinant ankG protein and fragments thereof of the present invention hold great promise in the generation of vaccines against AD and other Aβ associated diseases as well as in the use as a means for the detection of the risk of disease, diagnosis of disease, and disease progression and etiology. The following is a non-limiting list of Aβ associated diseases: Alzheimer's disease, Down's syndrome, Lewy body dementia, head trauma, dementia pugilistica, amyloid deposition associated with aging, mild cognitive impairment, vascular dementia, mixed dementia, hereditary cerebral hemorrhage with amyloidosis Dutch type and Icelandic type, glaucoma, Parkinson's disease, frontotemporal dementia, corticobasal degeneration, inclusion body myositis and cerebral amyloid angiopathy.

Concerning the detection or diagnosis of a neurological diseases, the present invention further relates to a method of diagnosing a neurological disorder, wherein the disorder is associated with Alzheimer's disease and/or wherein the disorder is associated with amyloid β (Aβ) pathology/amyloidosis *in vitro* comprising determining in a body fluid sample the presence of an anti-ankG autoantibody, wherein the presence or an elevated level of the antibody compared to the level in a control sample is indicative that an individual suffers from said disorder. Preferably the body fluid is cerebrospinal fluid or blood; see also Example 2, wherein significant occurrence of autoantibodies against ankG could be found in sera of AD in comparison to HCS patients. Furthermore cognitive decline over time was reduced in AD patients immunopositive against ankG.

Furthermore, the present invention provides a kit for use in the method for isolation an agent useful in the treatment of a neurological disorder or in the method of diagnosing a neurological disorder, as described above, said kit comprising ankG or a fragment thereof; APP or an ankG-binding fragment thereof; and/or an anti-ankG antibody.

In a further embodiment the present invention relates to a method of reducing brain Aβ pathology and lowering brain levels of Aβ comprising administering to a subject in need thereof a therapeutically effective amount of an agent as defined herein and above. For example, the agent used in said method of reducing brain Aβ pathology and lowering brain levels of Aβ according to the present invention may be any agent as defined herein and above capable of inducing an immune response in a subject, *e.g.,* recombinant ankG protein or a fragment, derivative or analog thereof introduced in the way of vaccination as shown in Example 3. Analogical, any agent capable of binding to ankG may be used in said method, *e.g.,* by passive immunization with antibodies against ankG as shown in Example 4.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness are given in Berks, TIBTECH 12 (1994), 352-364.

The above disclosure generally describes the present invention. Several documents are cited throughout the text of this specification. Full bibliographic citations may be found at the end of the specification immediately preceding the claims.

A more complete understanding can be obtained by reference to the following specific examples which are provided herein for purposes of illustration only

### EXAMPLES

The Examples which follow further illustrate the invention. Detailed descriptions of conventional methods, such as those employed herein can be found in the cited literature; see also "The Merck Manual of Diagnosis and Therapy" Seventeenth Ed. ed. by Beers and Berkow (Merck & Co., Inc., 2003).

The practice of the present invention will employ, unless otherwise indicated, conventional techniques of cell biology, cell culture, molecular biology, transgenic biology, microbiology, recombinant DNA, and immunology, which are within the skill of the art.

Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251.

### Material and Methods

### Human samples:

AD patients and HCS were recruited at the gerontopsychiatric department of the University of Zürich. AD cases used for sera analysis had an age at onset ≥ 65 years and a diagnosis of definite or probable AD according to the National Institute of Neurological and Communication Disorders and Stroke-Alzheimer's Disease and Related Disorders Association diagnostic criteria. HCS subjects were matched for age and sex. Cognitive assessment was recorded using MMSE. All subjects were followed up at yearly intervals for a period of up to 26 months with repeat MMSE on each occasion. The rate of cognitive decline for each group was based on the average slope of MMSE weighted for numbers of months of follow up. Human brains were obtained from the Kathleen Price Bryan Brain Bank from Duke University (USA). AD classification was assessed according to (52, 53).

### Tissue micro arrays (TMA).:

Immunohistochemical quantification of ankG expression was performed by TMA analysis from 288 tissue samples, each from AD and HCS (n=12). The selection of specimen and TMA construction were previously described (22). Primary antibodies were visualized using either a standard diaminobenzidine horseradish-peroxidase method for Aβ (Histofine simple Stain Max PO, Nichirei Bioscience, Nichirei, Japan) or alkaline phosphatase method for ankG staining (Histofine simple Stain Max AP, Nichirei). AnkG staining was assessed in 7 grades determined by the sum of a score for the % of immunopositive cells (0 = none, 1 = 1-10 %, 2 = 10-50 %, 3 = >50 %) and the intensity of the staining (0 = none, 1 = weak, 2 = moderate, 3 = strong, 4 = very strong).

### Animals.:

ArcAbeta mice were produced as described previously by (17). Tg2576 mice were bred from germinal lines produced as described previously (27). Non-transgenic littermates and APP deficient mice were used as controls when specified. All animals were housed 3-5 per cage and had access to food and water *ad libitum* under a 12-hour light/dark cycle. All *in vivo* experiments were performed with the approval of the Swiss veterinary council (BVET).

### Anaesthesia and perfusion.:

Mice were anaesthetized using a cocktail of 1.25 % ketamine and 0.25 % xylazine. After transcardial perfusion with ice-cold buffer PBS (10 mM Na₂HPO₄, 2.5 mM NaH₂PO₄, 150 mM NaCl, 3 mM KCl) one hemisphere was processed biochemically and the other one incubated in paraformaldehyde (PFA) 4 % in PBS for 4 hours at 4°C followed by incubation in sucrose 30 % in PBS for 24-36 h for immunohistochemistry purposes.

### Active ankG immunization.:

ArcAbeta mice or Tg2576 mice and their non-transgenic littermates were actively immunized with either 10 µg ankG protein in PBS + Freund's adjuvant complete (FA) (Sigma Aldrich F5881) or PBS + FA (control-immunized). Blood was drawn from the tail of each mouse before and during (every 4 weeks) immunization to obtain sera for antibody titre analysis. Immunization cocktails were injected subcutaneously between the scapulae. Three 4-weekly immunization re-boosts were performed with Freund's adjuvant incomplete instead of FA. At the end of the 3-months immunization period the mice were perfused. One hemisphere of the brain was fixed in paraformaldehyde solution for 4 hours at 4°C and subsequently put in a solution of 30 % sucrose at 4°C for cryoprotection for 24-36 hours for immunohistochemistry. The other hemisphere of the brain was processed for biochemical analysis.

### Neurological examination.:

Mini neurological examination was performed before, during and after each immunization as described in (17). Brief, all animals had normal fur appearance, no secretory signs, normal body postures and normal basic reflex, including the eye blink, pupillary, flexion and righting reflexes. All animals had age-appropriate body weight and no difference in muscular strength (grip strength) as measured with a spring scale.

### Cell lines.:

Human embryonic kidney cells (HEK 293, DSMZ, ACC 305) were cultured in Dulbecco's modified eagle medium (DMEM, Invitrogen #52100039) supplemented with 10% fetal calf serum (FCS) and penicillin/streptomycin (PenStrep, Invitrogen #10378-016) at 37°C, 5% CO₂, 95% humidity. SHY-5Y neuroblastoma cells were grown in DMEM/F12 supplemented with 20% fetal bovine serum. For differentiation, cells were sequentially treated for 5 days with 5 µM retinoic acid (Sigma, Buchs, Switzerland, Cat. No. 68268) and for 5 days with 20 ng/ml BDNF (Peprotech, London, UK, Cat. No. 450-02-10ug), with serum reduced to 2%, after which they were transfected identically to HEK293 cells. HEK293 cells expressing APP-Citrine were obtained and cultured as described in (54). HeLa cells stably expressing Swedish APP.

### siRNA silencing..

Silencing of the ankG gene (Validated siRNA cat. no. SI02780204, Qiagen AG, Switzerland), ankB gene (Validated siRNA cat. no. SI03031238, Qiagen AG, Switzerland) and negative control siRNA Alexa Fluor 488 (cat. no. 1022563), Qiagen AG, Switzerland) in differentiated SHY-5Y cell, HEK293, Citrine-HEK293, Swedish APP HEK293 and Swedish APP HeLa cells was carried out using the RNAi kit and RNAi nucleotides as per manufacturer's instructions (Qiagen). Silencing was repeated every 24 hours for 3 days followed by immunoblotting analysis. To inhibit γ-secretase activity, cells were cultured in the presence of 1 nM DAPT (Sigma Aldrich, Switzerland, Cat. No. 208255-80-5) as described in (54).

### Protein antigen arrays screening.:

The protein antigen arrays screening was custom-made by imaGenes GmbH, Berlin, Germany, containing 37,000 clones from a human fetal brain cDNA expression library as described in (22). Briefly, the protein arrays were utilized for high-throughput antibody screening and each clone was spotted in duplicates onto PVDF filter membranes (2 parts, each 26 cm X 26 cm in size) (Fischer Scientific, Cat. No. IPFL00010). For screening with sera antibodies, the protein array filters were first incubated with blocking buffer (3% non-fat milk in Tris-buffered saline [TBS]) for 2 hours and then incubated with the patient's sera (dilution 1:1000) for 14 hours. After extensive washing with TBS containing 0.05% Tween-20 and 0.5% Triton X-100 (TBS-T), filters were incubated with HRP-conjugated anti-human IgG (1:2000 diluted in blocking buffer) (Abcam, UK, Cat. No. ab 87422) for 1 hour. After several washings, the filters were developed by stabilized tetramethylbenzidine-blotting (TMB-blotting) substrate (Pierce, USA, Cat. No. 34021) for 5 minutes. Positive spots in duplicates indicated specific binding of sera antibodies to recombinant proteins. The corresponding expression clones were obtained from the RZPD and were cultured in LB Broth Base medium (Invitrogen Corp., Carlsbad, USA, Cat. No. 12780-029) supplemented with ampicillin (Teknova, Hollister, USA, and Cat. No. A9510). Their plasmids were isolated, and the cDNA inserts were sequenced for identification of the proteins. Immunoreactivity was assigned with the following score: (0 = none, 1 = weak, 2 moderate, 3 = strong, 4 = very strong). The strongest immunoreactive clones encoding for proteins expressed in the correct reading frame and which were immunopositive in at least 2 different sera were considered as positive hits.

### Image analysis.:

Immunohistochemical images were acquired on a Leica DM4000B microscope using an Olympus DP71 colour digital camera and newCAST software (Visiopharm, Copenhagen, Denmark). Image analysis was carried out with ImageJ image analysis software (56, 57). Quantification of the number of β-amyloid plaques and Iba-1 positive microglia was performed using the area measurement tool. For measurement of plaque diameter the particle count tool was used. The measurements were done on 10 sequential sections per mouse.

### WB analysis.:

Densitometric analyses of the WBs were done using ImageJ (Scion Corporation, Frederick, Maryland, US), (56, 57) or Tina Image analysis software (University of Manchester, UK; http://tina-vision.net). Mean optical intensities were plotted after standardization of intensities to loading controls.

### Statistical analysis.:

Data analysis, statistical evaluation and graphical representation of data were done using GraphPad Prism software (GraphPad Software, La Jolla, USA, http://www.graphpad.com/prism/Prism.htm). Mann Whitney test, t-test or Chi-square test was used to determine significance between the groups.

### Biochemical enrichment of Aβ.:

Frozen brain samples were homogenized in 1% TritonX-100 in buffer A (50 mM Tris-Cl, 100 mM NaCl, pH 8). The homogenate was centrifuged at 100000 g for 1 hour. The pellet obtained was resuspended in 2 % SDS and 2 mM EDTA in buffer A. The pellet obtained after centrifugation at 100000 g for 1 hour was enriched in Aβ. The protein concentration of each fraction was quantified by BCA assay kit (Pierce, Rockford, IL, USA Cat. No. 23225) and subjected to immunoblotting and Aβ ELISA. All buffers were used at 4°C with protease inhibitors (Roche Applied Science, Indianapolis, IN, USA, Cat. No. 11873580001).

### Exosome preparation:

Exosomes were isolated from HEK 293 cells. Briefly, cells from 3 to 6 T175 flasks (Sigma Aldrich, St. Louis, USA, Cat. No. CLS 3298) were cultured in DMEM with 10% FCS (Gibco, Invitrogen Corp., USA, Cat. No. 10108-157). A day before the exosome preparation, culture medium was replaced with AIM-V medium (Gibco, Invitrogen Corp., USA, Cat. No. 1205509). Culture supernatants of cells grown for 24 hrs in AIM-V medium were collected and spun at 300 *x g* for 10 min to remove cells. The supernatants were then sequentially centrifuged at 1200 x *g* for 20 mins, 10,000 *x g* for 30 mins, and 100,000 x g for 1 hour. The 100,000 x g pellet was washed with Phosphate buffer saline (PBS) and again spun at 100,000 *g* for 1 hour. The second 100,000 x g pellet (exosomal pellet) was resuspended in PBS. The pelleted fractions were then used for immunoblotting.

### Immunoblotting.:

Equal amounts of total protein were subjected to separation on 10-20% Tricine gels (Invitrogen, Basel, Switzerland), blotted on nitrocellulose membranes (0.45 µm; Biorad, Reinach, Switzerland, Cat. No. 162-0116) or Protran BA 79 cellulose-nitrate membranes (0.1 µm; Schleicher & Schuell, Dassel, Germany (Cat. No. 732-4018). The immunoblot was then incubated with primary antibodies (table S3) followed by incubation with HRP-tagged secondary antibodies.
Detection was performed using chemiluminescence visualized using ECL WB reagents (GE, Germany, Cat. No. RPN2109) or SuperSignal West Dura Extended Duration reagents (Pierce, Rockford, IL, USA, Cat. No. 34075) on BIOMAX films (GE, Germany, Cat. No. 25010141).

**Table S3. Antibodies and their application**

| **Antigen** | **Company** | **Product#** | **Application(s)** | **Antigen retrieval** |
|---|---|---|---|---|
| **ankG** | Santa Cruz | sc-28561 | WB, IP | NA |
| | Santa Cruz | sc-31778 | ELISA | NA |
| | Zymed® Laboratories | 33-8800 | IHC | HCl |
| | In-house produced monoclonal | NA | IHC | Protein K |
| **ankB** | Zymed® Laboratories | 33-3700 | WB | NA |
| **APP C-term.** | Sigma | A8717 | WB | NA |
| **APP N-term.** | Millipore | MAB348 | WB | NA |
| **APP / Aβ** | Covance / Signet | SIG-39300 | WB, IHC, IP | HCl: IHC. NA: WB & IP |
| | Dako | M0872 | IHC | NA |
| **Actin** | Abcam | ab6276 | WB | NA |
| **Tau** | Innogenetics | clone HT7 | WB | NA |
| **Neurofilament 200 kDa** | Millipore | AB1982 | IHC | HCl |
| **Caspr P6061** | Gift from Dr. E. Peles | | | |
| **TAP1** | (51) | | | |
| **Alix** | Biolegend | 634501 | WB | NA |
| **Calnexin** | Stressgen | SPA-865 | | |
| **Iba 1** | Wako | 1919741 | IHC | NA |

| | | | | |
|---|---|---|---|---|
| IHC = immunohistochemistry, IP = immunoprecipitation, WB = WB, NA = not applicable | | | | |

### Immunoprecipitation.:

Brain homogenates were lysed for 1 hour with lysis buffer, pH 7.5, containing 50 mM Tris-HCl (Sigma Aldrich, Cat. No. T5941) , 150 mM NaCl (Sigma Aldrich, Cat. No. S5886), 1 % Nonidet P-40 (Sigma Aldrich, Cat. No. 74385), 1 mM Na₂P₂O₇ (Sigma Aldrich, Cat. No. 221368), 1 mM NaF (Sigma Aldrich, Cat. No. 47072), 2 mM Na₃VO₄ (Sigma Aldrich, Cat. No. S6508), 0.1 mM PMSF (Sigma Aldrich, Cat. No. 7626), 2 mM EDTA (Sigma Aldrich, Cat. No. EDS-100G) and EDTA-free protease inhibitor cocktail (Roche, Roche Applied Science, Indianapolis, IN, USA, Cat. No. 11873580001). Lysates were precleared with non-specific IgG antibodies followed by protein A/G coated magnabeads (=Dynabeads® Invitrogen, USA, Cat. Nos. 100-01D, 100-03D), for 1 hour each. The precleared lysate was then incubated with indicated primary antibodies or non-specific IgG for 1 hour. Antibody complexes were then precipitated with beads applied for 1 hour. Beads were washed 5 times with lysis buffer and once with PBS and used for WB immunoblot analysis. All steps were carried out at 4°C. For every 1 mg/ml of total protein content of homogenate 3 µg antibodies were used

### Serum antibody titres.:

Blood samples were spun down at 3000 g for 10 minutes at 4°C to obtain sera for titre analysis. Anti-ankG antibodies in the serum of immunized mice were determined by ELISA techniques. Briefly, 0.5 µg/ml rat recombinant ankG (or 2 µg/ml BSA) in PBS were immobilized on the polyvinylchloride surface overnight at 4°C. Wells were washed five times for 5 minutes at room temperature (RT) with PBS containing 0.05 % Tween-20 (PBS-T) followed by blocking for 1 hour at RT with 2% BSA in PBS and subsequently incubation with immunized sera diluted 1:500 in PBS-T containing 3 % BSA for 2 hours at RT. Wells were washed and bound serum antibodies were detected with HRP-conjugated anti-mouse antibodies. The reaction was developed with 0.1% ABTS (Roche) in 100 mM acetate buffer, pH 5.0. The reaction was stopped with 100 mM sodium fluoride. OD was measured at 450 nm.

### Protein ligand binding assay.:

APP CTF-50 peptide (Calbiochem, EMD Chemicals, USA, Cat. No. 171545), Aβ40 and Aβ42 (Bachem AG, Weil am Rhein, Germany, Cat. Nos. H-1194, H1368) were coated on 96-well polyvinyl chloride plates at 0.5 µg/ml in PBS at 4°C overnight. Wells were then blocked for 1.5 hours with PBS containing 2% BSA and incubated for 2 hours at RT with rat recombinant ankG (0-2.5µg/ml) diluted in PBS-T. Washing steps were carried out between all incubations using PBS-T. Plates were incubated for 1.5 hours at RT with goat antibodies against ankG in PBS-T containing 2% BSA. Detection was as described before for serum titre analysis.

### ELISA quantification of Aβ levels.:

Concentrations of Aβ40 and Aβ42 from mice serum and SDS insoluble brain fractions were quantified using commercially available ELISA kits (Millipore, Billerica, USA, Cat. Nos. EZHS40, EZHS42) as per manufacturer's instructions. Sera were diluted 1:100 in PBS before use. Two to 4 µg at 25 µg/ml of total protein of SDS insoluble fractions in PBS was used per well.

### Immunohistochemistry.:

Fixed and cryoprotected hemibrains were cut in 30 µm thick slices at ~ -80°C using a microtome (Leica Jung HN40) and kept at -20°C in an anti-freeze solution (phosphate buffer 0.50 M in MilliQ water: ethyleneglycol: glycerol = 1.3:1:1) until staining was performed. All immunohistochemical stainings were executed using the free-floating method. Washing steps were carried out between all incubations using washing buffer (TBS pH 7.4 containing 0.2% Triton X-100 (Sigma-Aldrich, Cat. No. X100-5ML) at RT. Antigen retrieval was performed when required (Table S3) by incubating the slices in 1 M HCl at 65°C for 30 minutes. Slices were blocked for 1 hours at RT using blocking buffer (5.0% goat serum 5.0% donkey serum in washing buffer). Blocked slices were incubated overnight at 4°C with slight agitation in primary antibody incubation buffer (2.5% goat serum and 2.5% donkey serum in washing buffer). This was followed by secondary antibody incubations were carried out for 2 hours at RT. Slices were washed in washing buffer, mounted on chrom-gelatin-coated microscopy slides (Super-frost-plus, Menzel, Braunschweig, Germany, Cat. No. J1800AMNZ) and glass-covered using Hydromount® (National Diagnostics, Hull, UK, Cat. No. HS-106).

### Organotypic hippocampal slice cultures and Sindbis virus infection:

Organotypic hippocampal slice cultures were prepared and cultured according to (55). Briefly, six to seven day old arcAbeta transgenic and non-transgenic mice were decapitated, brains were removed, both hippocampi isolated and cut into 400 µm thick slices using tissue chopper. Slices were cultured on Millicell culture plate inserts (0.4 µm, Millipore, Bedford, MA, Cat. No PF187EN00) in six well plates containing 1 ml culture medium (46% minimum essential medium eagle with HEPES modification, 25% basal medium with earls modification, 25% heat-inactivated horse serum, 2 mM glutamine, 0.6% glucose, pH7.2). Culture plates were kept at 37°C in a humidified atmosphere containing 5% CO₂. Slices were kept in culture for 12 days before the experiments. Culture medium was exchanged every second to third day. On day 11 culture medium was replaced by low-serum Nb-N1 medium (94.5% Neurobasal medium, 0.5% heat-inactivated horse serum, 2 mM glutamine, 0.6% glucose, 1x N1 supplement, pH 7.2). On day 12 *in vitro* slice cultures were infected with Sindbis virus expressing EGFP using droplet method (29). For spine analysis, cultures were fixed at day 3 post infection within six-well plates. Slices were left attached to the culture plate membrane to preserve hippocampal structure and rinsed with PBS. Slices were then fixed with 4% paraformaldehyde in PBS containing 4% sucrose for 2h at 4°C. After washing with PBS, cultures were mounted and coverslipped. For analysis of effects of anti-ankG antibodies cultures were treated with 10µl antibody per 1ml culture medium for 1 week.

### Confocal imaging of fixed hippocampal slice cultures and analysis of spine density:

Confocal high-resolution imaging of spines was performed using TCS/SP2 Leica confocal laser scanning microscope with 63x objective (oil, NA: 1.4). Fragments of apical and basal dendrites of hippocampal CA1 and CA3 pyramidal neurons were imaged with voxel size of 0.058 x 0.058 x 0.25 µm in x-y-z direction. To determine spine density the length of the dendrite was measured and spines were counted as protrusions in x- and y-axes using NIH ImageJ software (56, 57).

### Example 1: The cytoskeletal adaptor protein ankG is redistributed into β-amyloid plaques in AD brains

To characterize the role of ankG in AD neuropathology, the distribution of ankG was studied in brains. Immunohistochemistry of AD hippocampal tissue revealed the presence of ankG in β-amyloid plaques (Fig. 1a). As expected (16), ankG was also present in neuronal cell bodies and axons in both AD and HCS brains. Interestingly, a similar pattern of ankG immunostaining was present in β-amyloid plaques and neurons of cortical sections from a transgenic mouse model for AD (Fig. 1b). This mouse model expresses human APP with the Swedish and Arctic familial AD-causing mutations (arcAbeta mice) (17). Expression of ankG and APP in dystrophic neurons was increased in older mice (Fig. 1b, lower panel) as compared to younger animals (Fig. 1b, upper panel), a finding correlating with disease progression. Immunohistochemical quantification of ankG in human frontal cortex showed an increase in ankG expression in AD versus HCS (Fig. 1c). AnkG co-distributed together with tau and Aβ on WB of SDS-insoluble protein fractions from human frontal cortex samples and increased levels of ankG in AD versus HCS were observed (Fig. 2a, 2b). As expected, tau protein levels were also increased (Fig. 2a) (18). Similar to human brain, ankG distribution was affected in hippocampal sections from 24 months old arcAbeta mice as compared to their non-transgenic littermates (Fig. 2c). In contrast, the staining patterns of neurofilament - a neuronal marker - showed intact gross hippocampal architecture (Fig. 2c). The redistribution of ankG into β-amyloid plaques also occurred in brains of 7 months old arcAbeta mice. These results suggest that redistribution of ankG from the neuronal cytoplasm to β-amyloid plaques could be due to a pathogenic mechanism occurring in AD rather than to a general effect of neurodegeneration or aging on brain architecture. It is known that cytosolic proteins such as heat shock proteins, PrP, Aβ and caspase-1 are released from the cell through exosomes which are small membrane vesicles involved in trafficking, cell-cell communication and immune response activation (19, 20). AnkG was found in exosomes purified from HEK293 cells (Fig. 2d), suggesting an exosome-mediated extracellular ankG release.

### Example 2: AnkG is associated with an immune response in AD

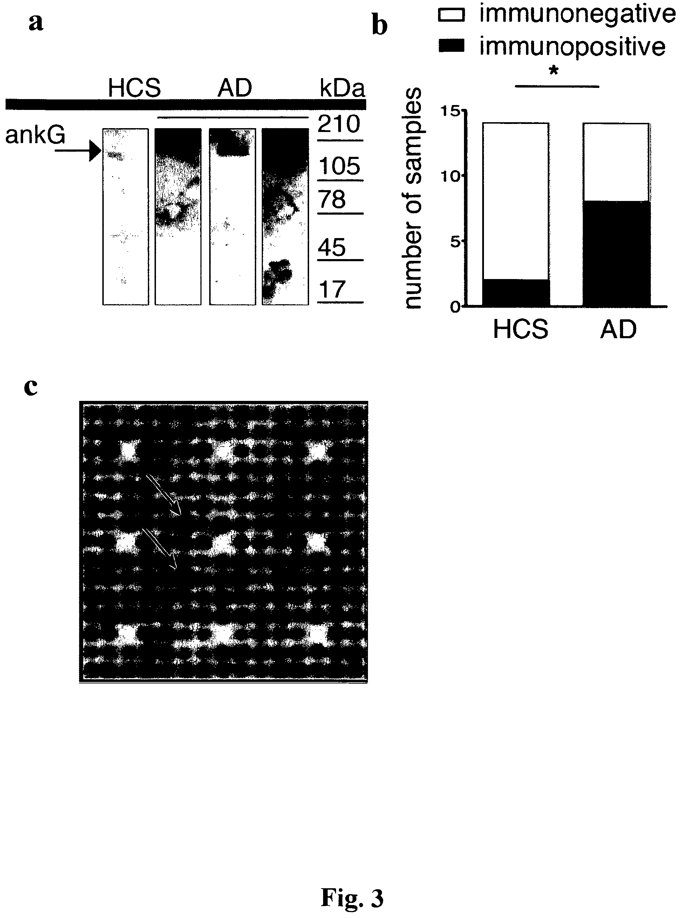

The abnormal extracellular and increased ankG expression pattern observed in AD brain and the presence of ankG in exosomes lead the inventors to hypothesize that ankG could provoke an immunoresponse in AD patients. To explore this possibility Western blot (WB) analyses was performed using purified ankG probed with sera from 14 AD patients and 14 HCS (table S1) (Fig. 3a, 3b).

**Table S1: Age and MMSE of the patient population**

| | **Age average** | **MMSE average** |
|---|---|---|
| **AD** | 74 ± 4.6 | 19.3 ± 6.1 |
| **HCS** | 70.4 ± 7.8 | 29.7 ± 0.4 |

Eight AD patients and only 2 of the HCS showed IgG against ankG (Fig. 1b). No IgM against ankG were found in these sera. The same AD sera did not cross-react with purified ankyrin B (ankB), a highly homologous protein of ankG (21) confirming that ankG antibodies found in AD patients were not the result of a general unspecific immunogenic reaction against members of the ankyrin family. The rate of cognitive decline over time in the AD population (n = 12 out of 14; 2 patients had only one cognitive assessment) was also assessed. Linear regression analysis of decline in the mini-mental score evaluation (MMSE) weighed for the number of follow-up months showed a significant reduced rate of cognitive decline in ankG-immunopositive versus immunonegative patients (table S2).

The limitation of this observation, however, is the population size and hence future studies with larger independent populations are required. To further prove the existence and specificity of antibodies against ankG in AD sera protein arrays generated from a human fetal brain cDNA expression library comprising 37,000 clones (22-24) were used and probed with an independent set of AD sera. In addition to ubiquitously expressed genes, this library contained a subset of proteins that are predominantly expressed in brain tissue. Sera from 15 AD patients and 10 age-matched healthy control subjects (HCS) were applied to separate protein arrays (Fig. 3c). A maximum of 15 clones were identified in each subject. Sequencing of immunopositive clones identified ankG among these proteins. These results suggest that ankG provokes an immunoresponse in AD patients with detectable antibodies in the sera

**Table S2: Comparison of cognitive score changes over time versus baseline in AD patients either immunopositive or immunonegative for sera antibodies against ankG**

| | **AnkG immunonegative AD patients (n=6)** | **AnkG immunopositive AD patients (n=6)** | **P value** |
|---|---|---|---|
| MMSE score at baseline ± SD | 23.5 ± 1.5 | 17.5 ± 6.55 | |
| Slope (points per each 6 months) ± SD | 0.40 ± 0.37 | 0.13 ± 0.29 | 0.04 |

### Example 3: Active immunization with ankG reduces β-amyloid pathology in vivo

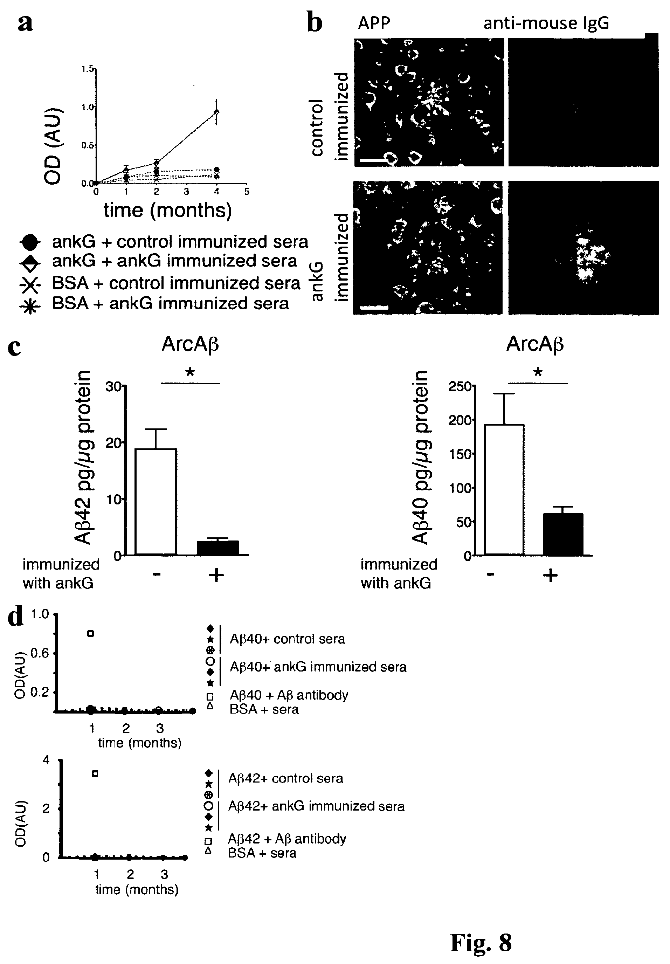

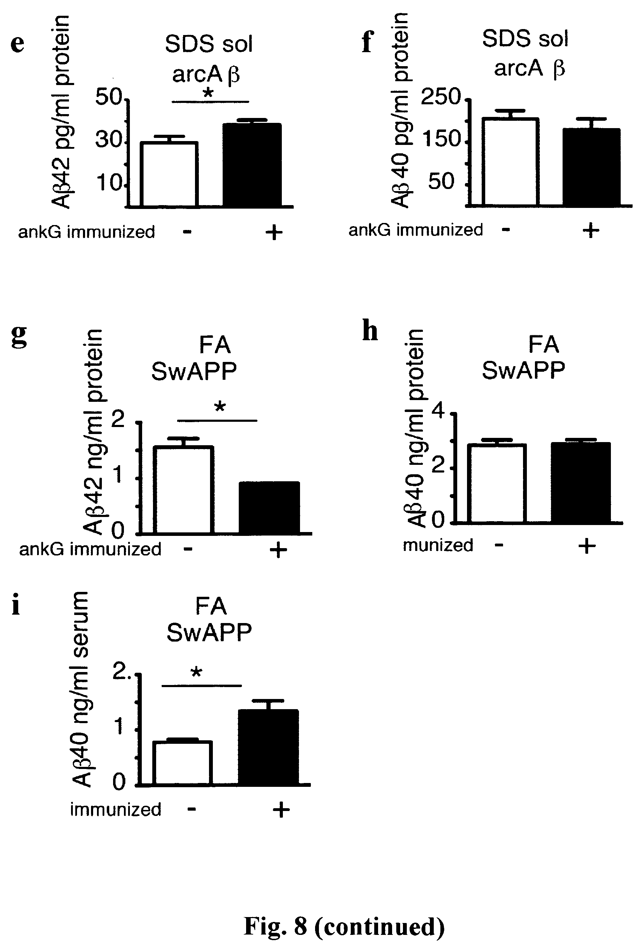

To study whether targeting ankG by immunotherapy affects the clearance of β-amyloid and to experimentally mimic the immune response observed in humans, 14 weeks old arcAbeta mice and their non-transgenic littermates were immunized. The mice received four monthly vaccinations of 10 µg recombinant ankG protein in Freund's adjuvant (FA) or PBS in FA as negative controls. Serum ELISA from ankG-immunized mice showed monthly increasing titers of ankG antibody (IgG) in comparison to controls (Fig. 8a). One month after the last immunization mice were sacrificed. Immunostaining against mouse IgGs in ankG-immunized arcAbeta mice showed their presence within the β-amyloid plaques as compared to controls, suggesting that antibodies against ankG can penetrate the blood-brain-barrier reaching ankG within plaques (Fig. 8b). AnkG-immunized and control-immunized mice did not show any obvious changes in phenotype during the immunization. Immunohistochemical analysis of brain sections revealed that active ankG immunization significantly reduced the number and size of β-amyloid plaques as compared to controls (Fig. 4a, 4b). WB analysis of brain SDS insoluble fractions showed reduced brain levels of Aβ in ankG-immunized mice as compared to controls (Fig. 4c). Expression levels of neurofilament and GAPDH were unchanged suggesting selectivity of ankG immunization for Aβ (Fig. 4 c). Quantitative ELISA of brain SDS insoluble fractions showed a significant reduction in Aβ40 and Aβ42 (Fig. 8c). In contrast to the reduced SDS-insoluble Aβ pool, the SDS-soluble Aβ42 fraction was significantly higher after ankG immunization suggesting ankG-immunization induced disaggregation of SDS-insoluble β-amyloid material with decreased insoluble Aβ40 and Aβ42 in the formic acid fraction followed by increased Aβ42 released into the soluble fraction (compare Fig. 8c versus Fig. 8g+h).

Serum ELISA from ankG-immunized arcAbeta mice did not show anti-Aβ antibodies, excluding the possibility of a general unspecific immune reaction against amyloid antigens including Aβ that could have caused Aβ clearance (Fig. 8d). As microglial cells can clear β-amyloid plaques by phagocytosis both in the presence and in the absence of antibodies against Aβ (25), it was investigated whether ankG molecules present in β-amyloid plaques were phagocytosed by microglia. Triple immunostainings for Iba1, a marker for activated microglia, APP/Aβ and ankG showed ankG immunoreactivity within activated microglia together with β-amyloid aggregates in both ankG-immunized and control-immunized arcAbeta brains (Fig. 4a). Notably, microglial density was significantly higher within the β-amyloid plaques of ankG-immunized as compared to controls (Fig. 4a, Fig. 9a). Antibody-mediated phagocytosis by microglia is a suggested mechanism for β-Amyloid plaque clearance *in vivo* (26). Hence, the results of the experiments performed in accordance with the present invention suggest that microglia participate in the clearance of β-amyloid plaques after active ankG immunization.

To further prove the potential of active immunization on β-amyloid plaque reduction 4 months old Tg2576 mice, another mouse model for AD harbouring the human "Swedish" APP mutation (27) were actively immunized. These mice display both amyloid pathology and memory deficits. The same immunization protocol as for the arcAbeta mice was applied. The mice were sacrificed at 7 months of age, an age at which only intraneuronal Aβ is seen in this mouse model. Serum ELISA of ankG-immunized mice showed monthly increasing titres of ankG antibody (IgG) only in 4 immunized mice out of 10. This variability in the immune response against ankG can be explained by their mixed genetic background. ELISA of formic acid extracted brain SDS insoluble fractions showed reduced brain levels of Aβ42 in the ankG-immunized mice versus controls (Fig. 9b and 8g). No significant reduction of Aβ40 was observed (Fig. 8h). Interestingly, higher levels of Aβ42 were found in the sera of these animals suggesting a higher clearance of Aβ42 versus controls (Fig. 9c and 8i). Such an plasma spike of Aβ42 was previously observed with some forms of Aβ immunotherapy that cleared soluble Aβ from the brain into the plasma via a so called peripheral "sink" mechanism (65).

### Example 4: Antibodies against ankG lower Aβ levels and β-induced spine loss in hippocampal cultures from arcAbeta mice.

To further understand the influence of anti-ankG antibodies observed *in vivo* it was investigated whether anti-ankG antibodies produced in arcAbeta mice after active immunization were capable of influencing Aβ levels. To this aim mouse monoclonal antibodies were produced using hybridoma cells (28). Antibodies (named mAbA) showing the strongest immunoreactivity against ankG by WB (Fig. 9d), ELISA and immunohistochemistry (data not shown) were applied to arcAbeta organotypic hippocampal slice cultures for 7 days. A reduction in Aβ40 and Aβ42 levels was observed as compared to untreated hippocampal slices using ELISA (Fig. 5a). A mouse antibody against bovine-herpes-virus-1 was used as control showing no effect on the Aβ levels. It is known that Aβ induces spine loss in hippocampal neurons (29). Therefore, the question was addressed whether anti-ankG antibodies could protect against spine loss. Sindbis virus-mediated expression of EGFP was used to allow visualization of neurons and spines (29). Hippocampal cultures from arcAbeta mice showed lower spine density as compared to non-transgenics (Fig. 5b, 5c). Treatment with mAbA abolished spine loss in arcAbeta hippocampal cultures (Fig. 5b, 5c). mAbA did not bind to Aβ40 and Aβ42 as measured by ELISA. No effect on spine density in non-transgenic cultures was observed proving a specific protective effect only on Aβ-induced spine loss (Fig. 5b, 5c).

### Example 5: APP but not Aβ interacts with ankG in the brain

Aβ is a hydrophobic peptide and thus the presence of ankG in β-amyloid plaques could be due to a non-specific interaction with hydrophobic ankG domains. Nevertheless, ELISA performed with recombinant ankG and synthetic fibrillar Aβ (Aβ40 and Aβ42) showed no interaction between ankG and Aβ (Fig. 10a). Similarly, Aβ was absent from ankG immunoprecipitates from human and mouse brain (Fig.6a, 6b). AnkG has been previously shown to induce clustering of adhesion molecules such as neurofascin-186 and Nr-CAM (2). APP is present on the neuronal membrane; therefore ankG could serve as its anchoring molecule. AnkG immunoprecipitation using human hippocampal homogenates showed that APP, but not Aβ, was present in the immunoprecipitated material (Fig. 6a); likewise APP co-immunoprecipitated with ankG in the reverse immunoprecipitation protocol (Fig. 6a). AnkB was not coimmunoprecipitated proving the specificity of this interaction (Fig. 6a). This specific ankG-APP interaction was also found in AD hippocampal homogenates (Fig. 6e) and in immunoprecipitates from arcAbeta and non-transgenic mice (Fig. 6b), suggesting a physiological interaction maintained also during brain β-amyloid pathology. APP-deficient mouse brains served as controls to eliminate the possibility of a non-specific interaction with IgG. Immunoprecipitation with species-specific IgG was used as negative control in all immunoprecipitation experiments (Fig. 6a). To be interacting partners two proteins have to be present in the same cellular compartment. AnkG is known to be enriched at the axonal initial segment (AIS) (2, 30) as is APP (Fig. 6c). To investigate whether the ankG-APP association is the result of a direct interaction, ELISA was performed using recombinant ankG and a synthetic peptide corresponding to the 50-amino acid cytoplasmatic fragment of APP (AICD50). It was found that ankG bound to AICD50 in a concentration-dependent manner but not to bovine serum albumin (Fig. 6d), suggesting an interaction between ankG and APP in the neuronal cytoplasm. The binding of ankG to APP and its presence in β-amyloid plaques could indicate that ankG stimulates amyloidogenic APP metabolism towards plaque formation.

### Example 6: AnkG is involved in APP metabolism

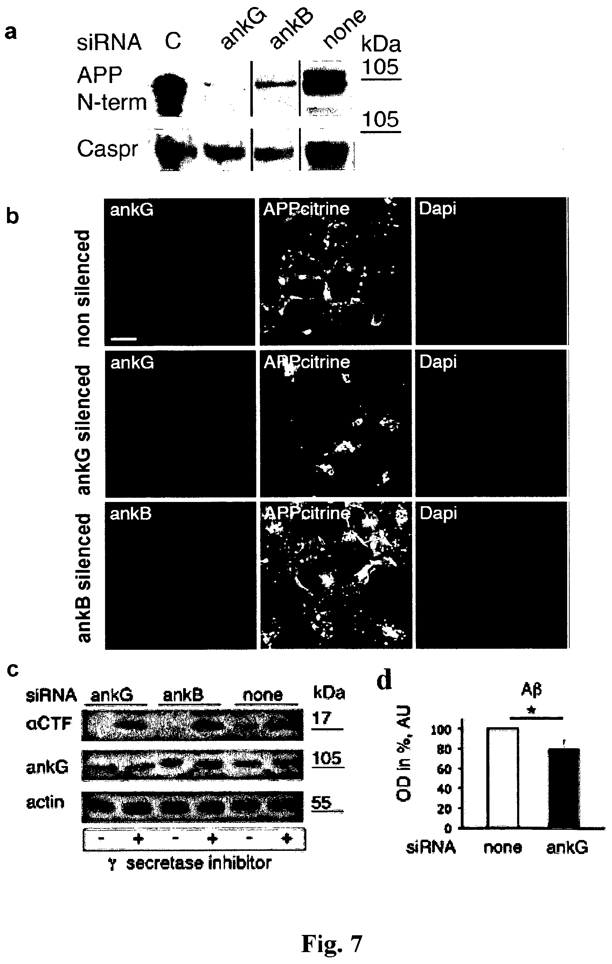

To characterize the function of the interaction between ankG and APP and to determine whether ankG recruits APP to the plasma membrane, membrane proteins were biotinylated after ankG RNAi-silencing in APP-overexpressing HEK293 cells. A decrease in cell surface-biotinylated APP was observed in ankG-silenced as compared to ankB-silenced and non-silenced cells (Fig. 7a). Similar findings were found using SH-SY5Y human neuroblastoma cells and APP-citrine overexpressing HEK293 cells (Fig. 6b). Loss of ankG protein expression did not affect total APP protein levels in the cell lysates (Fig. 7b and Fig. 10b). Under physiological conditions APP is mainly cleaved within the lumenal domain by α-secretase, resulting in shedding of nearly the entire ectodomain and generation of a membrane-tethered α-C-terminal fragment (α-CTF) (31). Upon ankG silencing, decreased amounts of α-CTF were found in lysates of cells treated with γ-secretase inhibitor when compared to γ-secretase-treated ankB-silenced or non-silenced cells (Fig. 7c, Fig. 10c). The γ-secretase inhibitor was required to increase the detectable amount of α-CTF, the substrate of the γ-secretase. Similar results were observed using HEK293 cells. Similar results were also observed using SH-SY5Y cells (Fig. 10e). Brain levels of APP were decreased in ankG-immunized arcAbeta mice, with less APP in the membrane-containing SDS soluble fraction (Fig. 10f, 10g) adding *in vivo* evidence for the possibility that ankG contributes to the trafficking and subsequent processing of APP with resulting impact on Aβ generation. This possibility is also supported by the ELISA results of culture media of ankG-silenced Swedish APP over-expressing HeLa cells showing a reduction in Aβ40 levels as compared to controls (Fig. 7d), with levels of Aβ42 levels below detection. Moreover, ankG silencing reduced total Aβ as shown in cell lysates of HEK293 cells over-expressing Swedish APP (Fig. 10d). Together, these results indicate a role for the ankG-APP interaction in the cellular trafficking and subsequent processing of APP with a significant role in its amyloidogenic processing that ultimately results in Aβ formation and the subsequent assembly of neuropathologic aggregation products.

### Example 7: AnkG-immunization is not neurotoxic and does not interfere with known physiological functions of ankG in neurotransmission

To investigate whether ankG immunization influences known physiological functions of ankG in neurotransmission or causes any neurotoxicity, immunized and non-immunized wildtype and arcAbeta mice were subjected to Y-maze testing to measure hippocampal-dependent memory. There was no difference in behaviour in ankG-immunized wild type mice (see Fig. 11a, 11b) suggesting that ankG immunization was neither neurotoxic nor interfering with the known physiological functions of ankG in neurotransmission. Taken together with the absence of ankG antibodies in neuronal cell bodies and axons (see Fig. 8b), these findings support the idea that by ankG-reactive antibodies preferentially target extracellular ankG including ankG bound to β-amyloid plaques rather than intraneuronal ankG. Despite that no significant difference in performance between ankG-immunized and non-immunized arcAbeta mice was found, it is prudent to presume an positive effect of immunization or prolonged treatment with anti-ankG antibodies on cognitive functions of AD patients in respect of other effects of such treatment, as described above, e.g., observed protective effect of anti-ankG antibodies in hippocampal slice cultures reduction (see, Fig. 5a+b), reduced size and plaque load of β-amyloid-plaques (see, Fig. 4a+b) and higher clearance of β-amyloid (see, Fig. 4c) as discussed in further detail below.

### DISCUSSION

AnkG is a cytoplasmic adaptor protein involved in the anchoring and assembly of voltage-gated ion channels in the axonal initial segment of neurons throughout the brain (2, 30), and genetic variations in the ankG-encoding gene ANK3 on chromosome 10q21 encoding ankG are a major risk factor for bipolar disorder (60). The results of this study establish that ankG in AD brain is abnormally expressed acquiring immunogenic properties triggering a humoral immune response with the generation of ankG-reactive IgG antibodies, and they point to a novel role of ankG in the neuropathology of AD. This is in line with the cytoskeletal pathological rearrangements known to occur in neurodegenerative disorders which affect physiological events including intracellular trafficking (13). In fact, data obtained in accordance with the present invention show that in AD, ankG - a soluble neuronal cytoplasmic protein under normal conditions - is partly relocated extracellularly to β-amyloid plaques, hence becoming an inducer of immune responses. The presence of ankG in exosomes can explain the release of this intracytoplasmic protein in the extracellular compartment and therefore its immunogenicity. This was accompanied by the presence of ankG antibodies in AD sera, wherein the frequency of ankG immunoreactive sera was higher in patients with AD as compared to aged-matched HCS. Most strikingly, cognitive decline in ankG-immunopositive AD patients was found to be lower as compared to immunonegative AD patients suggesting that ankG antibodies could be neuroprotective in humans. Serum antibodies against ankG were accompanied by extracellular accumulations of ankG in AD brains, and cortical levels of ankG were significantly higher in AD than in HCS. The partial relocalization of ankG from the neuronal cytoplasm to β-amyloid plaques in the neuropil of AD brains is possibly mediated through exosomal release as suggested by the presence of ankG in exosomes. Extracellular ankG, in particular β-amyloid plaque-associated ankG could serve as an immunogen triggering the observed humoral immune responses in AD patients as well as in aged human subjects who are known to deposit brain β-amyloid long before the onset of the clinical signs of AD. By analogy, humoral immune responses against β-amyloid are well established in AD, MCI and non-demented aged subjects. Active immunization with ankG reduced both β-amyloid plaque load and brain concentrations of Aβ in two independent transgenic mouse models (arcAbeta and Tg2576 ("SwAPP") mice). Reduced β-Amyloid plaque load and brain concentrations of insoluble aggregates of Aβ₄₀ and Aβ₄₂ in formic acid fractions of arcAbeta mice was accompanied by increased levels of soluble Aβ₄₂ in SDS soluble fractions (see Fig. 8e), compatible with immunization-induced disaggregation of insoluble material and resulting in the release of Aβ₄₂ into the soluble pool. Aβ₄₂ has synaptotoxic activities mediated through interactions with nicotinic receptors and NMDA receptors (69) and transient increases may occur during disaggregation of fibrillar β-amyloid followed by microglial phagocytosis and clearance. It is possible that the initial disaggregation phase of β-amyloid clearance is not accompanied by immediate beneficial effects on neuronal function and behaviour, which may be expected to follow clearance of the toxic peptides and regeneration of neuronal structures (61). In experiments performed in accordance with the present invention no changes were observed in behavioural tests during the time interval of ankG immunization experiments, neither in transgenic nor in wild-type mice. This finding resembles the observation of progressive dementia in AD patients during Aβ₄₂ immunization which clearly lowered brain β**-**amyloid plaque load (61, 70). To address more directly the roles of ankG-reactive antibodies on the morphology of neurons subjected to Aβ-related toxicity, dendritic spine morphology in organotypic brain slices prepared from transgenic mice expressing human mutant APP was analysed. The results of these experiments showed that ankG antibodies almost completely rescued the Aβ-related spine loss in brain slices from transgenic as compared to non-transgenic wild-type littermates. This was associated with decreased levels of Aβ within the organotypic slice preparation. Because dendritic spines are known to react very sensitively to Aβ, these data suggest Aβ reduction as a potential mechanism for the beneficial effects of ankG antibodies on dendritic spines in arcAbeta mouse brains slices. Data presented hereinabove showed that microglial cells are involved in phagocytosis of ankG present within β-amyloid plaques, possibly via Fc receptor-mediated phagocytosis of IgG reacting with ankG bound to β-amyloid. While ankG immunization showed the potential to reduce and clear β-amyloid to protect neurons from Aβ-related damage, it is necessary to determine whether these potentially beneficial effects can be achieved without disrupting ankG's physiological functions in anchoring voltage-gated ion channels and adhesion molecules to the axonal cytoskeleton. This would require, however, specific neutralization of ankG in its physiological cytoplasmic localization, a compartment that is unlikely targeted by extracellular antibodies that may be internalized into luminal endosomal compartments with limited capabilities to escape into the cytoplasm. Experiments described hereinabove support the unlikeliness of such disruption of ankG's physiological functions as active immunization with ankG in mice did not lead to any obvious side effects.

Studies on passive and active immunization with Aβ proceeded rapidly to clinical trials, at which stage 6 % of the patients developed meningoencephalitis (32, 33). Since then there has been a search for other immunotherapeutic candidates in AD. Recent findings showed that active immunization against the phosphorylated tau epitope in the P301L tangle mouse model reduces aggregated tau in the brain and slows progression of the tangle-related phenotype (34, 35), and alpha-synuclein immunization reduced the related pathology in transgenic mouse models (71). However, at least in respect of tau this immunization led to neurological defects caused by adverse immunogenic reactions (35). Although preliminary, active immunization with ankG in mice did not lead to any obvious side effects. AnkG immunotherapy shows promise for clearing β-amyloid. In order to achieve immunization with ankG interfering with APP trafficking and β-amyloid clearance without disrupting physiological functions of ankG one approach could be a specific immunotherapeutic subcellular targeting of ankG with no cross-reactivity against endogenously functioning ankG. Previous studies have found autoantibodies in AD patients against spectrin, glial fibrillary acidic protein, myelin basic protein and aldolase, raising the question as to whether these antibodies are neurotoxic or neuroprotective (36-40). The study performed in accordance with the present invention shows for the first time that auto-antibodies against ankG could have neuroprotective potential by lowering Aβ toxicity most likely modulating APP metabolism. In addition to its genetic association with bipolar disorder (68), *ANK3,* the gene encoding AnkG was found to be one of the 23 functional candidate genes associated with late onset AD (14, 41, 64). A perspective for ankG as a potential biomarker for AD is strongly supported by the present data. If immunogenicity of ankG is linked not only to AD pathology but also to disease progression, the presence of anti-ankG antibodies could serve as a biomarker. Such a possibility is addressed in the present study although but requires further investigation. Furthermore, if immunogenicity of ankG is not only linked to AD pathology but also to bipolar disorder, the presence of anti-ankG antibodies may influence the clinical course of this cerebral affection as well, also requiring further studies to address this possibility.

Bennett and co-workers showed that ankG is involved in the targeting of several membrane proteins to specialized domains of the plasma membrane (6, 42-44). AnkG is also involved in the assembly of molecules at the nodes of Ranvier and the AIS of neurons (44, 45-47). In accordance with the present invention it was be shown for the first time that ankG, by interacting directly with APP, contributes to its membrane localization and processing. The loss of surface localization of APP in the absence of ankG opens the possibility that APP is targeted by ankG to a specific micro-domain of the plasma membrane. Precise surface targeting of APP is a prerequisite for the proposed role of APP in synapse formation and stability, as well as functioning as a cell surface receptor (48). Abnormal expression of ankG as seen in AD brains taken together with the role of ankG in APP membrane targeting and Aβ production suggests an alteration of these ankG-mediated events in AD. Since it is known that altered trafficking of APP is directly linked to aberrant proteolytic processing of APP and Aβ production (49) anti-ankG antibodies could interfere with APP processing, contributing to reduction of Aβ production and therefore β-amyloid plaque formation. Previous studies describe auto-antibodies in AD patients against spectrin, glial fibrillary acidic protein, myelin basic protein and aldolase (36-40). Data presented herein of reduced cognitive decline in AD patients with positive serum titers of ankG-reactive antibodies provides evidence for a protective potential of such antibodies in AD. Should similar antibodies be present in IVIG preparations consisting of pooled IgG fractions derived from a large number of human donors, these may contribute to some of the beneficial effects of IVIG observed in clinical trials.

In accordance with the present invention it was also shown that microglial cells are involved in phagocytosis of ankG molecules present within β-amyloid plaques. It is speculated that microglia are recruited to phagocytose β-amyloid plaques by the activation of their Fc receptors through antibodies directed against brain antigens (50) which could also be the case for ankG antibodies. In conclusion, the experiments performed in accordance with the present invention demonstrate that ankG constitutes a β-amyloid-related antigen, and that AD patients generate an antibody immune response against it. This study further supports a therapeutic role for ankG antibodies in AD as underlined by the significant reduction in β-amyloid plaques via anti-ankG antibodies *in vivo* as well as a decrease in Aβ-induced spine loss *ex vivo.* The present data also show a role for ankG in cellular processing of APP.

### References

1. Mohler, P.J., Bennett, V. 2005. Defects in ankyrin-based cellular pathways in metazoan physiology. Front. Biosci. 10: 2832-2840.
2. Dzhashiashvili, Y., Zhang, Y., Galinska, J., Lam, I., Grumet, M., Salzer J.L. 2007. Nodes of Ranvier and axon initial segments are ankyrin G-dependent domains that assemble by distinct mechanisms. J. Cell Biol. 177:857-870.
3. Tuvia, S., Garver, T.D., Bennett, V. 1997. The phosphorylation state of the FIGQY tyrosine of neurofascin determines ankyrin-binding activity and patterns of cell segregation. Proc. Natl. Acad Sci. USA 94:12957-12962.
4. Zhou, D., Lambert, S., Malen, P.L., Carpenter, S., Boland, L.M., Bennett, V. 1998. AnkyrinG is required for clustering of voltage-gated Na channels at axon Initial segments and for normal action potential firing. J. Cell. Biol. 143:1295-1304.
5. Song, A.H., Wang, D., Chen, G., Li, Y., Luo, J., Duan, S., Poo, M.M. 2009. A selective filter for cytoplasmic transport at the axon initial segment. Cell 6:1148-1160.
6. Kizhatil, K., Baker, S.A., Arshavsky, V.Y., Bennett, V. 2009. Ankyrin-G promotes cyclic nucleotide-gated channel transport to rod photoreceptor sensory cilia. Science 323:1614-1617.
7. Fache, M-P., Moussif, A., Fernandes, F., Giraud, P., Garrido, J.J., Dargent, B. 2004. Endocytic elimination and domain-selective tethering constitute a potential mechanism of protein segregation at the axonal initial segment. J. Cell Biol. 166:571-578.
8. Shaw, A.S., Filbert, E.L. 2009. Scaffold proteins and immune-cell signalling. Nat. Rev. Immunol. 9:47-56.
9. Bennett, V., Healy, J. 2009. Membrane domains based on ankyrin and spectrin associated with cell-cell interactions. Cold Spring Harb. Perspect. Biol. 1:a003012.
10. Rasband, M.N. 2010. The axon initial segment and the maintenance of neuronal polarity. Nat. Rev. Neurosci. 11:552-562.
11. Sanchez-Ponce, D., Tapia, M., Munoz, A., Garrido, J.J. 2008. New role of IKKα / β phosphorylated IκBα in axon outgrowth and axon initial segment development. Mol. Cell. Neurosci. 37:832-844.
12. Sethi, G., Tergaonkar, V. 2009. Potential pharmacological control of the NF-κB pathway. Trends Pharmacol. Sci. 30:313-321.
13. Bamburg, J.R., Bloom, G.S. 2009. Cytoskeletal pathologies of Alzheimer disease. Cell. Mot. Cytoskel. 66:635-649.
14. Bertram, L. et al. 2000. Evidence for genetic linkage of Alzheimer's disease to chromosome 10q. Science 290:2302-2303.
15. Wang, G., Zhang, Y., Chen, B., Cheng, J. 2003. Preliminary studies on Alzheimer's disease using cDNA microarrays. Mechan. Agreing Dev. 124:115-124.
16. Zhang, X., and Bennett, V. 1998. Restriction of 480/270-kD ankyrin G to axon proximal segments requires multiple ankyrin G-specific domains. J. Cell. Biol. 142:1571-1581.
17. Knobloch, M., Konietzko, U., Krebs, D.C., Nitsch, R.M. 2007. Intracellular Abeta and cognitive deficits precede beta-amyloid deposition in transgenic arcAbeta mice. Neurobiol. Aging 28:1297-1306.
18. Grundke-Iqbal, I., Iqbal, K., Quinlan, M., Tung, Y.C., Zaidi, M.S., Wisniewski, H.M. 1986. Microtubule-associated protein tau. A component of Alzheimer paired helical filaments. J. Biol. Chem. 261:6084-6089.
19. Aguzzi, A., Rajendran, L. 2009. The transcellular spread of cytosolic amyloids, prions, and prionoids. Neuron 64: 783-790.
20. Qazi, K.R., Gehrmann, U., Domange Jordö, E., Karlsson, M.C., Gabrielsson, S. 2009. Antigen-loaded exosomes alone induce Thl-type memory through a B-cell-dependent mechanism. Blood 113:2637-2683.
21. Mohler, P.J. 2006. Ankyrins and human disease: what the electrophysiologist should know. J. Cardiovasc. Electrophysiol. 17:1153-1159.
22. Cepok, S., et al. 2005. Identification of Epstein-Barr virus proteins as putative targets of the immune response in multiple sclerosis. J. Clin. Invest. 115:1352-1360.
23. Lalive, P.H., Menge, T., Barman, I., Cree, B.A., Genain, C.P. 2006. Identification of new serum autoantibodies in neuromyelitis optica using protein microarrays. Neurology 67:176-177.
24. Horn, S., et al. 2006. Profiling humoral autoimmune repertoire of dilated cardiomyopathy (DCM) patients and development of a disease-associated protein chip. Proteomics 6:605-613.
25. Morgan, D. 2009. The role of microglia in antibody-mediated clearance of amyloid-beta from the brain. CNS Neurol. Disord. Drug Targets 8:7-15.
26. Koenigsknecht-Talboo, J. et al. 2008. Rapid microglial response around amyloid pathology after systemic anti-Abeta antibody administration in PDAPP mice. J. Neurosci. 28:14156-14164.
27. Hsiao, K. et al. 1996. Correlative memory deficits, Abeta elevation, and amyloid plaques in transgenic mice. Science 274:99-103.
28. Koehler, G., Milstein, C. 1975. Continuous cultures of fused cells secreting antibody of predefined specificity. Nature 256:495-497.
29. Tackenberg, C., Brandt, R. 2009. Divergent pathways mediate spine alterations and cell death induced by amyloid-beta, wild-type tau, and R406W tau. J. Neurosci. 29:14439-14450.
30. Komada, M., Soriano, P. 2002. [Beta]IV-spectrin regulates sodium channel clustering through ankyrin-G at axon initial segments and nodes of Ranvier. J. Cell Biol. 156:337-348.
31. Thinakaran, G., Koo, E.H. 2008. Amyloid precursor protein trafficking, processing, and function. J. Biol. Chem. 283:29615-29619.
32. Hock, C. et al. 2003. Antibodies against beta-amyloid slow cognitive decline in Alzheimer's disease. Neuron 38:547-554.
33. Orgogozo, J.M. et al. 2003. Subacute meningoencephalitis in a subset of patients with AD after Abeta42 immunization. Neurology 61:46-54.
34. Asuni, A.A., Boutajangout, A., Quartermain, D., and Sigurdsson, E.M. 2007. Immunotherapy targeting pathological tau conformers in a tangle mouse model reduces brain pathology with associated functional improvements. J. Neurosci. 27:9115-9129.
35. Rosenmann H. et al. 2006. Tauopathy-like abnormalities and neurologic deficits in mice immunized with neuronal tau protein. Arch. Neurol. 63:1459-1467.
36. Singh, V.K., Yang, Y.Y., Singh, E.A. 1992. Immunoblot detection of antibodies to myelin basic protein in Alzheimer's disease patients. Neurosci. Lett. 147:25-28.
37. Ounanian, A., Guilbert, B., Renversez, J.C., Seigneurin, J.M., Avrameas, S. 1990. Antibodies to viral antigens, xenoantigens, and autoantigens in Alzheimer's disease. J. Clin. Lab. Anal. 4:367-375.
38. Mor, F., Izak, M., Cohen, I.R. 2005. Identification of aldolase as a target antigen in Alzheimer's disease. J. Immunol. 175:3439-3445.
39. Mecocci, P. et al. 1995. Serum anti-GFAP and anti-S100 autoantibodies in brain aging, Alzheimer's disease and vascular dementia. J. Neuroimmunol. 57:165-170.
40. Kellner, A., Matschke, J., Bernreuther C., Moch, H., Ferrer, I., Glatzel, M. 2009. Autoantibodies against beta-amyloid are common in Alzheimer's disease and help control plaque burden. Ann. Neurol. 65:24-31.
41. Morgan, A.R. et al. 2007. Association studies of 23 positional/functional candidate genes on chromosome 10 in late-onset Alzheimer's disease. Am. J. Med. Genet. B. Neuropsychiatr. Genet. 144B:762-770.
42. Mohler, P.J. et al. 2004. Nav1.5 E1053K mutation causing Brugada syndrome blocks binding to ankyrin-G and expression of Nav1.5 on the surface of cardiomyocytes. Proc. Natl.Acad. Sci. USA 101:17533-17538.
43. Kizhatil, K., Davis, J.Q., Davis, L., Hoffman, J., Hogan, B.L., Bennett, V. 2007. Ankyrin-G is a molecular partner of E-cadherin in epithelial cells and early embryos. J. Biol. Chem. 282:26552-26561.
44. Kizhatil, K., Yoon, W., Mohler, P.J., Davis, L.H., Hoffman, J., Bennett, V. 2007. Ankyrin-G and beta2-spectrin collaborate in biogenesis of lateral membrane of human bronchial epithelial cells. J. Biol. Chem. 282:2029-2037.
45. Jenkins, S.M., Bennett, V. 2001. Ankyrin-G coordinates assembly of the spectrin-based membrane skeleton, voltage-gated sodium channels, and L1 CAMs at Purkinje neuron initial segments. J. Cell Biol. 155:739-746.
46. Jenkins, S.M., Bennett, V. 2002. Developing nodes of Ranvier are defined by ankyrin-G clustering and are independent of paranodal axoglial adhesion. Proc. Natl. Acad. Sci . USA 99:2303-2308.
47. Pan, Z., et al. 2006. A common ankyrin-G-based mechanism retains KCNQ and NaV channels at electrically active domains of the axon. J. Neurosci. 26:2599-2613.
48. Turner, P.R., O'Connor, K., Tate, W.P., Abraham, W.C. 2003. Roles of amyloid precursor protein and its fragments in regulating neural activity, plasticity and memory. Prog. Neurobiol. 70:1-32.
49. Rajendran, L., et al. 2008. Efficient inhibition of the Alzheimer's disease beta-secretase by membrane targeting. Science 320:520-523.
50. Diamond, B., Huerta, P.T., Mina-Osorio, P., Kowal, C., and Volpe, B.T. 2009. Losing your nerves? Maybe it's the antibodies. Nat. Rev. Immunol. 9:449-456.
51. Biscaro, B., Lindvall, O., Hock, C., Ekdahl, C.T., Nitsch, R.M. 2009. Abeta immunotherapy protects morphology and survival of adult-born neurons indoubly transgenic APP/PS1 mice. J. Neurosci. 29:14108-14119.
52. Mirra, S.S. et al. 1991. The Consortium to Establish a Registry for Alzheimer's Disease (CERAD). Part II. Standardization of the neuropathologic assessment of Alzheimer's disease. Neurology 41:479-486.
53. Braak, H., Braak, E. 1991. Neuropathological stageing of Alzheimer-related changes. Acta Neuropathol. 82:239-259.
54. Goodger, Z.V., Rajendran, L., Trutzel, A., Kohli, B.M., Nitsch, R.M., Konietzko, U. 2009. Nuclear signaling by the APP intracellular domain occurs predominantly through the amyloidogenic processing pathway. J. Cell Sci. 122:3703-3714.
55. Stoppini, L., Buchs, P.A., Muller, D. 1991. A simple method for organotypic cultures of nervous tissue. J. Neurosci. Methods 37:173-182.
56. Rasband, W.S., ImageJ, U. S. National Institutes of Health, Bethesda, Maryland, USA, http://rsb.info.nih.gov/ij/, 1997-2009.
57. Abramoff, M.D., Magelhaes, P.J., Ram, S.J. "Image Processing with ImageJ". Biophotonics International, volume 11, issue 7, pp. 36-42, 2004.
58. Togo, T. et al. Occurrence of T cells in the brain of Alzheimer's disease and other neurological diseases. J Neuroimmunol 124:83-92 (2002).
59. Akiyama, H. et al. Inflammation and Alzheimer's disease Neurobiol Aging 21:383-421(2000).
60. Nitsch, R. M. and Hock, C. Targeting beta-amyloid pathology in Alzheimer's disease with Abeta immunotherapy. Neurotherapeutics. 5:415-20 (2008).
61. Serrano Pozo A. et al. Beneficial effect of human anti-Aβ active immunization (AN1792) on amyloid deposition and neurite morphology. Program No. 825.5. 2009 Neuroscience Meeting Planner (2009).
62. Bennett V. and Healy, J. Organizing the fluid membrane bilayer: diseases linked to spectrin and ankyrin. Trends Mol Med. 14:28-36 (2008).
63. Paez-Gonzalez P. et al. Ank3-dependent SVZ niche assembly is required for the continued production of new neuron. Neuron. 71:61-75 (2011).
64. Myers A. et al. Susceptibility locus for Alzheimer's disease on chromosome 10. Science 290:2304-2305 (2000).
65. Zlokovic, B.V. eta al. Low-density lipoprotein receptor-related protein-1: a serial clearance homeostatic mechanism controlling Alzheimer's amyloid β-peptide elimination from the brain. J Neurochem. 115:1077-89.
66. Emmanouilidou, E. et al. Cell-produced alpha-synuclein is secreted in a calcium-dependent manner by exosomes and impacts neuronal survival. J Neurosci. 30:6838-51(2010)
67. Thinakaran, G., and Koo, E.H. Amyloid precursor protein trafficking, processing, and function. J Biol Chem 283:29615-29619 (2008).
68. Ferreira, M.A. et al. Collaborative genome-wide association analysis supports a role for ANK3 and CACNA1C in bipolar disorder. Nat Genet. 40:1056-1058 (2008)
69. Bordji, K., Becerril-Ortega, J., Buisson, A. Synapses, NMDA receptor activity and neuronal Abeta production in Alzheimer's disease. Rev Neurosci. 22:285-94. (2011).
70. Holmes, C. et al. Long-term effects of Abeta42 immunisation in Alzheimer's disease: follow-up of a randomised, placebo-controlled phase I trial. Lancet. 372(9634):216-223 (2008).
71. Masliah, E. et al. Passive immunization reduces behavioral and neuropathological deficits in an alpha-synuclein transgenic model of Lewy body disease. PLoS One. 6:e19338 (2011).
72. Crawley, J.N., What's wrong with my mouse? Behavioral phenotyping of transgenic and knockout mice. Hoboken, New Jersey: John Wiley & Sons, Inc. (2007).
73. Shahani, N. et al. Tau aggregation and progressive neuronal degeneration in the absence of changes in spine density and morphology after targeted expression of Alzheimer's disease-relevant tau constructs in organotypic hippocampal slices. J Neurosci 31:6103-6114 (2006).

## Claims

1. An agent selected from a recombinant ankG protein or a fragment thereof formulated as a vaccine which is capable of inducing autoantibodies against ankG for use in the treatment, amelioration or prevention of a neurological disorder, preferably wherein the disorder is associated with Alzheimer's disease, more preferably wherein the disorder is associated with amyloid β (Aβ) pathology/amyloidosis.

2. The agent for use according to claim 1, which is capable of interfering with the interaction of ankG and amyloid precursor protein (APP).

3. A method of diagnosing a neurological disorder as defined in claim 1 *in vitro* comprising determining in a body fluid sample the presence of an anti-ankG autoantibody, wherein the presence or an elevated level of the antibody compared to the level in a control sample is indicative that an individual suffers from said disorder, preferably wherein the body fluid is cerebrospinal fluid or blood.

4. Use of a kit in a method of claim 3, said kit comprising ankG or a fragment thereof; APP or an ankG-binding fragment thereof; and/or an anti-ankG antibody.

5. An agent as defined claims 1 or 2 for use in reducing brain Aβ pathology and lowering brain levels of Aβ.

## Patentansprüche

1. Wirkstoff ausgewählt aus einem rekombinanten ankG Protein oder einem Fragment davon, formuliert als Impfstoff der imstande ist Autoantikörper gegen ankG zu induzieren zur Verwendung in der Behandlung, Linderung oder Vorbeugung einer neurologischen Erkrankung, wobei vorzugsweise die Erkrankung mit Alzheimer-Krankheit verbunden ist, wobei weiter bevorzugt die Erkrankung mit β-Amyloid (Aβ) Pathologie/Amyloidose verbunden ist.

2. Wirkstoff zur Verwendung nach Anspruch 1, der imstande ist die Interaktion von ankG und dem Amyloid-Vorläuferprotein (APP) zu stören.

3. Verfahren zur Diagnose einer neurologischen Krankheit wie in Anspruch 1 definiert *in vitro,* umfassend das Bestimmen des Vorhandenseins eines Anti-ankG-Autoantikörpers in einer Körperflüssigkeitsprobe, wobei das Vorhandensein oder ein erhöhter Spiegel des Antikörpers im Vergleich zu dem Spiegel in einer Kontrollprobe bezeichnend dafür ist, dass ein Individuum unter dieser Krankheit leidet, wobei vorzugsweise die Körperflüssigkeit Rückenmarksflüssigkeit oder Blut ist.

4. Verwendung eines Kits in einem Verfahren nach Anspruch 3, dieses Kit umfassend ankG oder ein Fragment davon; APP oder ein ankG-bindendes Fragment davon; und/oder ein Anti-ankG-Antikörper.

5. Wirkstoff wie in Ansprüchen 1 oder 2 definiert zur Verwendung in der Reduzierung von Gehirn-Aβ-Pathologie und Senkung des Spiegels von Aβ im Gehirn.

## Revendications

1. Agent choisi parmi une protéine ankG recombinante ou un fragment de celle-ci, formulé sous la forme d'un vaccin qui est capable d'induire des autoanticorps dirigés contre ankG, pour son utilisation dans le traitement, l'amélioration ou la prévention d'un trouble neurologique, de préférence dans lequel le trouble est associé à la maladie d'Alzheimer, de manière davantage préférée dans lequel le trouble est associé à une pathologie liée l'amyloïde β (Aβ)/à une amylose.

2. Agent pour son utilisation selon la revendication 1, qui est capable d'interférer avec l'interaction d'ankG et la protéine précurseur d'amyloïde (APP).

3. Méthode de diagnostic *in vitro* d'un trouble neurologique tel que défini dans la revendication 1, comprenant la détermination, dans un échantillon de fluide corporel, de la présence d'un autoanticorps anti-ankG, dans lequel la présence ou un taux élevé de l'anticorps par rapport au taux dans un échantillon témoin indique qu'un individu souffre dudit trouble, de préférence dans lequel le fluide corporel est le liquide céphalo-rachidien ou le sang.

4. Utilisation d'un kit dans une méthode selon la revendication 3, ledit kit comprenant ankG ou un fragment de celle-ci ; APP ou un fragment de celle-ci se liant à ankG ; et/ou un anticorps anti-ankG.

5. Agent tel que défini dans la revendication 1 ou 2, pour son utilisation dans la réduction d'une pathologie cérébrale liée à Aβ et la diminution des taux d'Aβ dans le cerveau.
